(19) Europäisches Patentamt
European Patent Office
Office européen des brevets

(11) EP 1 158 297 A1

(12) # EUROPEAN PATENT APPLICATION
published in accordance with Art. 158(3) EPC

(43) Date of publication:
**28.11.2001 Bulletin 2001/48**

(21) Application number: **00902091.8**

(22) Date of filing: **04.02.2000**

(51) Int Cl.7: **G01N 33/50**, G01N 33/15, A61K 31/59, G01N 33/82

(86) International application number:
**PCT/JP00/00614**

(87) International publication number:
**WO 00/49403 (24.08.2000 Gazette 2000/34)**

(84) Designated Contracting States:
**AT BE CH CY DE DK ES FI FR GB GR IE IT LI LU MC NL PT SE**
Designated Extension States:
**AL LT LV MK RO SI**

(30) Priority: **18.02.1999 JP 4009099**

(71) Applicant: **CHUGAI SEIYAKU KABUSHIKI KAISHA**
**Tokyo, 115-8543 (JP)**

(72) Inventors:
- **UCHIYAMA, Yasushi**
  **Chugai Seiyaku Kabushiki Kaisha**
  **Gotenba-shi, Shizuoka 412-8513 (JP)**
- **SATO, Hideki Chugai Seiyaku Kabushiki Kaisha**
  **Gotenba-shi, Shizuoka 412-8513 (JP)**
- **KAKE, Takei Chugai Seiyaku Kabushiki Kaisha**
  **Gotenba-shi, Shizuoka 412-8513 (JP)**

(74) Representative: **VOSSIUS & PARTNER**
**Siebertstrasse 4**
**81675 München (DE)**

(54) **METHOD FOR SCREENING COMPOUND HAVING AFFINITY FOR VITAMIN D RECEPTOR**

(57) The object of the present invention is to provide a method for screening a vitamin D derivative which has a limited or no blood calcium increasing action, while retaining its useful physiological activities.

According to the present invention, there is provided a method for screening a compound having an affinity for a vitamin D receptor, comprising measuring a vitamin D receptor-associated, VDRE-mediated transcription promoting activity of a test compound and a vitamin D receptor-associated inhibitory action of the test compound against activity of a transcription factor; and selecting a compound which is more potent in inhibitory action against the activity of the transcription factor than in VDRE-mediated transcription promoting activity.

EP 1 158 297 A1

SEQUENCE LISTING

<110> Chugai Seiyaku Kabushiki Kaisha
<120> A method for screening a compound having an affinity with a vitamin D receptor

<130> F 2300 EP

<150> Japan 99/040090
<151> 1999-2-18

<160> 3

<210> 1
<211> 9
<212> DNA
<213> Homo sapiens
<400> 1
atgagtcag 9

<210> 2
<211> 20
<212> DNA
<213> Homo sapiens
<400> 2
cagaggggac ttttccgaga 20

<210> 3
<211> 15
<212> DNA
<213> Mus musculus
<400> 3
ggttcacgag gttca 15

## Description

### TECHNICAL FIELD

The present invention relates to a novel method for screening a vitamin D derivative, which causes few adverse reactions while retaining useful physiological activities. More particularly, the invention relates to a screening method which includes measuring a test compound's (vitamin D receptor-associated) transcription promoting activity mediated by a vitamin D responsive element (hereinafter referred to as "VDRE") and the test compound's (vitamin D receptor-associated) inhibitory activity against a transcription regulating activity of other particular transcription factors. The present invention also relates to a compound which is selected using the screening method, and to a drug containing the compound.

### BACKGROUND ART

Active vitamin $D_3$ derivatives, such as 1α,25-dihydroxyvitamin $D_3$ or 1α-hydroxyvitamin $D_3$, are already widely used in clinical settings as drugs for treatment of metabolic bone diseases such as osteoporosis. These active vitamin $D_3$ derivatives are known to exhibit physiological actions via vitamin D receptors. Vitamin D receptors are present not only in tissues such as the small intestine, bones, kidneys and parathyroid glands, but also in various cells, including cells of the immune system and malignant tumor cells. Thus, active vitamin $D_3$ derivatives are known to have various-physiological activities, such as (1) the action of regulating calcium and bone metabolism; (2) inhibition of proliferation of malignant tumor cells, epidermal cells and epithelial cells; and (3) regulation of immune system cells.

However, an adverse reaction which is known to arise from treatment with active vitamin $D_3$ derivatives is hypercalcemia, which poses problems with clinical use. If a blood calcium increasing action can be inhibited or eliminated selectively from the physiological actions that active vitamin $D_3$ derivatives possess, drugs can be produced which are excellent for the treatment of metabolic bone diseases. Such drugs will be novel therapeutic agents created on the basis of their varying physiological activities, and will cause few adverse reactions.

To date, the blood calcium increasing action of active vitamin $D_3$ derivatives has been demonstrated to occur by the following mechanism: An active vitamin $D_3$ derivative binds to a vitamin D receptor to form a complex. The complex binds to VDRE, which is present in a promoter region of a gene for a protein involved in calcium absorption in the small intestine (for example, calbindin-D in charge of calcium transport in the intestinal epithelial cell, or Ca-ATPase having the function of passing absorbed calcium on to the blood vessel), to induce the expression of the gene. As a result, the expression of these proteins causes calcium absorption from the intestinal tract.

Useful physiological activities of active vitamin $D_3$ derivatives have also been considered to appear when the complexes of active vitamin $D_3$ derivatives and vitamin D receptors bind to VDRE. Thus, screening a compound, which has a limited or no blood calcium increasing action, while retaining its useful physiological activities, has been considered extremely difficult.

Recently, the existence of a gene expression regulation mechanism by a vitamin D receptor based on the activity of inhibiting the transcriptional regulatory function of other transcription factors has been confirmed in transcription factors such as AP-1 complex or NF-κB family (see Xiao-Peng Yu et al., Proc. Natl. Acad. Sci. USA, Vol. 92, pp. 10990-10994, 1995; Hanna Harant et al., Eur. J. Biochem. 250, 63-71 (1997); Daniele D'Ambrosio et al., J. Clin. Invest., Vol. 101, No. 1, (1998), 252-262; Yasuo Kuroki et al., Journal of Cellular Physiology 164:459-464 (1995); Terri L. Towers et al., The Journal of Biological Chemistry, Vol. 273, No. 17, pp. 10338-10348 (1998); Atsuko Takeuchi et al., The Journal of Immunology, 1998, 160:209-218; and Iris Alroy et al., Molecular and Cellular Biology, Vol. 15, No. 10, pp. 5789-5799 (1995)). This mechanism of regulation of gene expression by a vitamin D receptor based on the action of inhibiting the activity of a transcription factor has been suggested to be totally different from the conventionally known mechanism of gene expression regulation based on binding of a vitamin D receptor to VDRE; and to be a mode of action which is not mediated by binding to VDRE.

However, there have been no reports so far of attempts to separate, in vitamin D derivatives, vitamin D receptor-associated, VDRE-mediated activity of promoting transcription and vitamin D receptor-associated inhibitory action on the activity of a transcription factor; with such inhibitory action being confirmed in transcription factors such as AP-1 complex or NF-κB family.

### DISCLOSURE OF THE INVENTION

It is an object of the present invention to provide a method for screening a vitamin D derivative which has a limited or no blood calcium increasing action, while retaining its useful physiological activities.

It is another object of the present invention to provide selectively a vitamin D derivative, which has a limited or no blood calcium increasing action, while retaining its useful physiological activities, by using the above screening

method.

It is still another object of the present invention to provide a drug for treatment of a disease in which the action of a transcription factor is involved the activity of which can be inhibited by a vitamin D receptor, the drug containing the above-mentioned vitamin D derivative.

It is a further object of the present invention to provide a kit for performing the screening method according to the present invention.

The inventors of the present invention confirmed initially that a vitamin D derivative inhibits the expression of a reporter gene without mediation by VDRE. This effect was confirmed by means of a reporter gene assay using a reporter gene vector having a binding site of AP-1 complex in a promoter region, and which was free from VDRE, and a reporter gene assay using a reporter gene vector having a binding site of NF-κB family in a promoter region, also free from VDRE.

Based on this finding, the inventors have-speculated that the blood calcium increasing action of vitamin D derivatives can be reduced or eliminated while retaining their therapeutic effect against diseases, the onset or progression of which diseases relates to the activation of a transcription factor such as AP-1 complex or NF-κB family. This effect is achieved by limiting or eliminating the vitamin D receptor-associated, VDRE-mediated transcription-promoting activity of vitamin D derivatives, while simultaneously retaining or enhancing the vitamin D receptor-associated transcription-inhibitory actions of the vitamin D derivatives on other transcription factors, which actions are typified by a non-VDRE mediated inhibitory action on the activity of AP-1 complex or NF-κB family. In other words, the adverse blood calcium increasing action of vitamin D derivatives can be limited or eliminated, while their therapeutic effect against diseases is retained, by increasing selectivity towards an action inhibitory of the activity of a transcription factor, relative to a VDRE-mediated transcription promoting activity.

In osteoporosis, for example, excessive formation of osteoclasts is believed to be causative of onset. It is known that formation of osteoclasts is inhibited in mice deficient in the function of AP-1 complex or NF-κB family as a transcription factor. Thus, it is expected that inhibition of the activity of these transcription factors will have a therapeutic effect against osteoporosis. It has been assumed that the therapeutic effect of active vitamin D derivatives against osteoporosis can be attributed to a mode of action whereby a vitamin D receptor binds to VDRE to promote transcription of a gene. However, it is now considered that this therapeutic effect is caused via a vitamin D. receptor-associated action which inhibits the activity of a transcription factor such as AP-1 complex or NF-κB family.

This applies not only to vitamin D derivatives, but also to substances having an affinity for vitamin D receptors.

Based on these new concepts, the inventors conducted investigations in an effort to develop a screening system to be used in searching for vitamin D derivatives which have few adverse effects, while retaining their useful physiological activities. As a result, the inventors measured the VDRE-mediated transcription promoting activity of vitamin D derivatives (the mechanism causing a blood calcium increasing action) by means of a reporter gene assay using a reporter gene vector having VDRE. The inventors also measured the inhibitory action (physiological activity) of vitamin D derivatives against the activity of a transcription factor, typified by inhibitory activity against AP-1 complex or NF-KB family, by means of a reporter gene assay using a reporter gene vector having a binding site of AP-1 complex in a promoter region and free from VDRE, and by means of a reporter gene assay using a reporter gene vector having a binding site of NF-KB family in a promoter region and free from VDRE. From these studies, the inventors developed an experimental screening system in which a derivative was found which has a higher selectivity in inhibiting the action of the transcription factor, as compared with 1$\alpha$,25-dihydroxyvitamin $D_3$.

In fact, by the screening system of the present invention there was found a vitamin D derivative having very high selectivity in inhibiting the action of the transcription factor, as compared with 1$\alpha$,25-dihydroxyvitamin $D_3$. The usefulness of this derivative as a drug was evaluated using an osteoporosis model. The derivative was confirmed to have activity as a drug useful for the treatment of osteoporosis, without affecting the urinary calcium excretion level, and without increasing the blood calcium level. The present invention was accomplished based on these findings.

According to the present invention, there is provided a method for screening a compound having an affinity for a vitamin D receptor, which comprises measuring the vitamin D receptor-associated, VDRE-mediated transcription promoting activity of a test compound, and the vitamin D receptor-associated inhibitory action of the test compound on the activity of a transcription factor, and selecting a compound which more strongly inhibits the activity of the transcription factor, relative to the promotion of VDRE-mediated transcription activity.

In the present invention, the compound having the affinity for the vitamin D receptor is preferably a vitamin D derivative.

In the present invention, a preferred example of the transcription factor is AP-1 complex or NF-κB family.

In a mode of the present invention, the VDRE-mediated transcription promoting activity is measured by means of a reporter gene assay system.

In a mode of the present invention, the action which inhibits the activity of the transcription factor is measured by means of a reporter gene assay system.

In a mode of the present invention, a test compound and a standard compound are measured for VDRE-mediated transcription promoting activity and inhibitory action of the activity of the transcription factor. The test compound should satisfy the following equation: (a relative value of the inhibitory action of the test compound against the activity of the transcription factor as compared with that of the standard compound)/(a relative value of the VDRE-mediated transcription promoting activity of the test compound as compared with that of the standard compound) > 1; particularly preferably a test compound satisfying the following equation: (a relative value of the inhibitory action of the test compound against the activity of the transcription factor as compared with that of the standard compound)/(a relative value of the VDRE-mediated transcription promoting activity of the test compound as compared with that of the standard compound) ≥ 10 is selected as the compound which is more potent in inhibitory action against the activity of the transcription factor relative to the VDRE-mediated transcription promoting activity.

In a preferred mode of the present invention, the standard compound is 1α,25-dihydroxyvitamin $D_3$.

According to another aspect of the present invention, there is provided a compound having an affinity for a vitamin D receptor, characterized in that the compound is selected by using the above-described screening method and the inhibitory action of the compound against the activity of the transcription factor is stronger relative to the VDRE-mediated transcription promoting activity of the compound.

The selected compound having the affinity for the vitamin D receptor is preferably a vitamin D derivative.

According to still another aspect of the present invention, there is provided a therapeutic drug for a transcription-factor-related disease. The activity of the transcription factor is inhibited by the vitamin D receptor, and the therapeutic drug contains a compound which selected using the screening method of the present invention and which has an affinity for the vitamin D receptor.

A nonrestrictive example of the transcription-factor-related disease is a metabolic bone disease, especially osteoporosis.

A preferred example of the transcription factor involved in the transcription-factor-related disease targeted by the therapeutic drug of the present invention is AP-1 complex or NF-κB family.

The therapeutic drug of the present invention can be used as a vitamin D receptor-mediated inhibitor of the activity of AP-1 complex, or as a vitamin D receptor-mediated inhibitor of the activity of NF-κB family.

According to a further aspect of the present invention, there is provided a drug for treatment of osteoporosis, containing as an active ingredient a compound satisfying the following equation: (a relative value of the inhibitory action of the test compound against the activity of the transcription factor as compared with that of the standard compound) /(a relative value of the VDRE-mediated transcription promoting activity of the test compound as compared with that of the standard compound) > 1, more preferably ≥ 10, when the VDRE-mediated transcription promoting activity and the inhibitory action against the activity of the transcription factor are measured using the screening method of the present invention.

The compound is preferably a vitamin D derivative represented by the following general formula (1):

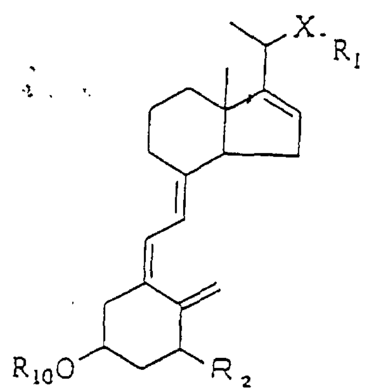

(1)

wherein X represents an oxygen atom or a sulfur atom, $R_1$ represents a saturated or unsaturated aliphatic hydrocarbon group optionally substituted by a hydroxyl group or a protected hydroxyl group or a $-COR_{12}$ group (wherein $R_{12}$ is an alkyl group, an aryl group or an alkoxy group), $R_2$ represents $-OR_9$ or a hydrogen atom, and $R_9$ and $R_{10}$ are the same or different and each represent a hydrogen atom or a protecting group. Further preferably, $R_2$ is $-OR_9$.

Preferably, $R_1$ is a saturated aliphatic $C_{1-15}$hydrocarbon group optionally substituted by a hydroxyl group or a unsaturated aliphatic $C_{2-15}$hydrocarbon group optionally substituted by a hydroxyl group. Further preferably, $R_1$ is a group (2)

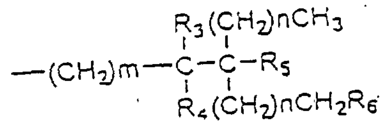

(2)

where $R_3$ and $R_4$ are the same or different and each represent a hydrogen atom or a hydroxyl group, or together have an oxygen atom to represent =O, provided that $R_3$ and $R_4$ are not hydroxyl groups at the same time, $R_5$ and $R_6$ each represent a hydrogen atom or a hydroxyl group, provided that $R_6$ is not a hydroxyl group at the same time as $R_3$ or $R_4$, m denotes an integer of 1 to 4, and n denotes an integer of 0 to 2,
or $R_1$ is a group (3)

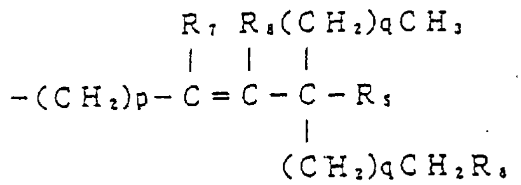

(3)

where $R_5$ and $R_6$ are the same or different and each represent a hydrogen atom or a hydroxyl group, $R_7$ and $R_8$ each represent a hydrogen atom or together represent a covalent bond, p denotes an integer of 1 to 3, and q denotes an integer of 0 to 2. Even more preferably, $R_1$ is a 3-hydroxy-3-methylbutyl group.

In the vitamin D derivative represented by the general formula (1), the 20-position may be in an S- configuration or an R-configuration.

Examples of the vitamin D derivative represented by the general formula (1) are 1,3-dihydroxy-20-(3-hydroxy-3-methylbutylthio)-9,10-secopregna-5,7,10(19),16-tetraene, 1α,3β-dihydroxy-20(S)-(3-hydroxy-3-methylbutylthio)-9,10-secopregna-5,7,10(19),16-tetraene, 1α,3β-dihydroxy-20(R)-(3-hydroxy-3-methylbutylthio)-9,10-secopregna-5,7,10(19),16-tetraene, 1α,3β-dihydroxy-20(R)-((E)-4-hydroxy-4-methyl-2-pentenylthio)-9,10-secopregna-5,7,10(19),16-tetraene, 1α,3β-dihydroxy-20(R)-((E)-4-ethyl-4-hydroxy-2-hexenylthio)-9,10-secopregna-5,7,10(19),16-tetraene, 1α,3β-dihydroxy-20(S)-(2-hydroxy-2-methylpropylthio)-9,10-secopregna-5,7,10(19),16-tetraene, 1α,3β-dihydroxy-20(R)-(2-hydroxy-2-methylpropylthio)-9,10-secopregna-5,7,10(19),16-tetraene, 1α,3β-dihydroxy-20(S)-{2(S)-hydroxy-3-methylbutyloxy}-9,10-secopregna-5,7,10(19),16-tetraene, 1α,3β-dihydroxy-20(S)-{2(R)-hydroxy-3-methylbutyloxy}-9,10-secopregna-5,7,10(19),16-tetraene, 1α,3β-dihydroxy-20(S)-(2-ethyl-2-hydroxybutylthio)-9,10-secopregna-5,7,10(19),16-tetraene, 1α,3β-dihydroxy-20(R)-(2-ethyl-2-hydroxybutylthio)-9,10-secopregna-5,7,10(19),16-tetraene, 1α,3β-dihydroxy-20(R)-(4-ethyl-4-hydroxy-2-hexynyloxy)-9,10-secopregna-5,7,10(19),16-tetraene and 1α,3β-dihydroxy-20(R)-{3-ethyl-2(S)-hydroxypentylthio}-9,10-secopregna-5,7,10(19),16-tetraene.

According to a still further aspect of of the present invention, there is provided a kit for performing the screening method of the present invention, which includes:

(a) a vector for evaluating the inhibitory action against activity of the transcription factor, the vector containing a binding sequence of the transcription factor and a reporter gene;
(b) a vector for evaluating the VDRE-mediated transcription promoting activity, the vector containing a VDRE sequence and a reporter gene;
(c) if desired, a vitamin D receptor expression vector; and
(d) a reagent for detecting a product of the reporter gene.

BRIEF DESCRIPTION OF THE DRAWINGS

FIG. 1 shows the results of screening of VDRE-mediated transcription promoting activity and inhibitory activity against AP-1 complex.

FIG. 2 shows the results of screening of VDRE-mediated transcription promoting activity and inhibitory activity against NF-κB family.

FIGS. 3(a) to 3(f) are graphs showing the actions of vitamin D derivatives (administered subcutaneously) on urinary calcium excretion, blood calcium concentration and bone mineral density.

FIGS. 4(a) to 4(c) are graphs showing the actions of vitamin D derivatives (administered orally) on urinary calcium excretion, blood calcium concentration and bone mineral density.

FIGS. 5(a) to 5(d) are graphs showing the actions of vitamin D derivatives (administered orally) on blood Ca value, urinary deoxypyridinoline (Dpyr) excretion, bone mineral density and bone strength.

## BEST MODE FOR CARRYING OUT THE INVENTION

Hereinafter, more specific modes of the present invention and methods for carrying out the present invention will be described, but the invention is not restricted by the following description.

Substances to be screened by the method of the present invention are not restricted and may be any compounds having an affinity for a vitamin D receptor. Generally, the substances are vitamin D derivatives, especially vitamin $D_3$ derivatives, and particularly preferably active vitamin $D_3$ derivatives. Nonrestrictive examples of the vitamin D derivatives are vitamin D derivatives expressed by the aforementioned general formula (1).

The terms used in the present invention have the following meanings unless otherwise defined:

The saturated aliphatic hydrocarbon group generally refers to a straight chain or branched chain alkyl group having 1 to 15 carbon atoms. Its examples include a methyl group, an ethyl group, an n-propyl group, an i-propyl group, an n-butyl group, an s-butyl group, an i-butyl group, a t-butyl group, a pentyl group, a hexyl group, a heptyl group, an octyl group, a nonyl group and a decanyl group. Preferred examples are a 3-methylbutyl group, a 3-ethylpentyl group, a 4-methylpentyl group, a 3-(n-propyl)hexyl group, a 4-ethylhexyl group, a 5-methylhexyl group, a 6-methylheptyl group, a 5-ethylheptyl group and a 4-(n-propyl)heptyl group. More preferred examples are a 3-methylbutyl group, a 3-ethylpentyl group and a 4-methylpentyl group.

The unsaturated aliphatic hydrocarbon group generally refers to a straight chain or branched chain alkenyl group or alkynyl group having 2 to 15 carbon atoms. Examples include a 2-propenyl group, a 2-butenyl group, a 3-butenyl group, a 2-pentenyl group, a 3-pentenyl group, a 4-pentenyl group, a 2-hexenyl group, a 3-hexenyl group, a 4-hexenyl group, a 5-hexenyl group, a 2-heptenyl group, a 3-heptenyl group, a 4-heptenyl group, a 5-heptenyl group, a 6-heptenyl group, a 2-propynyl group, a 2-butynyl group, a 3-butynyl group, a 2-pentynyl group, a 3-pentynyl group, a 4-pentynyl group, a 2-hexynyl group, a 3-hexynyl group, a 4-hexynyl group, a 5-hexynyl group, a 2-heptynyl group, a 3-heptynyl group, a 4-heptynyl group, a 5-heptynyl group and a 6- heptynyl group. Any of the hydrogen atoms thereof may be substituted by one or more of the aforementioned alkyl groups and cis- or trans-form may be present with respect to the double bond. Preferred examples are a 4-methyl-2-pentynyl group, a 4-ethyl-2-hexynyl group, a 4-methyl-2-pentenyl group and a 4-ethyl-2-hexenyl group.

The saturated or unsaturated aliphatic hydrocarbon group optionally substituted with a hydroxyl group refers to the above-mentioned saturated or unsaturated hydrocarbon group, any of whose hydrogen atom may be substituted by one or more hydroxyl groups. Examples of the number of the substituting hydroxyl groups are 0, 1, 2 and 3, of which preferred is 1 or 2, more preferably 1. Specific examples are not only the aforementioned aliphatic hydrocarbon groups, but also saturated aliphatic hydrocarbon groups, such as a 2-hydroxy-2-methylpropyl group, a 3-hydroxy-2-methylpropyl group, a 2,3-dihydroxy-2-methylpropyl group, a 2-ethyl-2-hydroxybutyl group, a 2-ethyl-3-hydroxybutyl group, a 2-ethyl-2,3-dihydroxybutyl group, a 2-hydroxy-2-(n-propyl)pentyl group, a 3-hydroxy-2-(n-propyl)pentyl group, a 2,3-dihydroxy-2-(n-propyl)pentyl group, a 2-hydroxy-3-methylbutyl group, a 3-hydroxy-3-methylbutyl group, a 4-hydroxy-3-methylbutyl group, a 2,3-dihydroxy-3-methylbutyl group, a 2,4-dihydroxy-3-methylbutyl group, a 3,4-dihydroxy-3-methylbutyl group, a 3-ethyl-2-hydroxypentyl group, a 3-ethyl-3-hydroxypentyl group, a 3-ethyl-4-hydroxypentyl group, a 3-ethyl-2,3-dihydroxypentyl group, a 3-ethyl-2,4-dihydroxypentyl group, a 3-ethyl-3,4-dihydroxypentyl group, a 2-hydroxy-3-(n-propyl)hexyl group, a 3-hydroxy-3-(n-propyl)hexyl group, a 4-hydroxy-3-(n-propyl)hexyl group, a 2,3-dihydroxy-3-(n-propyl)hexyl group, a 2,4-dihydroxy-3-(n-propyl)hexyl group, a 3,4-dihydroxy-3-(n-propyl)hexyl group, a 3-hydroxy-4-methylpentyl group, a 4-hydroxy-4-methylpentyl group, a 5-hydroxy-4-methylpentyl group, a 3,4-dihydroxy-4- methylpentyl group, a 3,5-dihydroxy-4-methylpentyl group, a 4,5-dihydroxy-4-methylpentyl group, a 4-ethyl-3-hydroxyhexyl group, a 4-ethyl-4-hydroxyhexyl group, a 4-ethyl-5-hydroxyhexyl group, a 4-ethyl-3,4-dihydroxyhexyl group, a 4-ethyl-3,5-dihydroxyhexyl group, a 4-ethyl-4,5-dihydroxyhexyl group, a 3-hydroxy-4-(n-propyl)heptyl group, a 4-hydroxy-4-(n-propyl)heptyl group, a 5-hydroxy-4-(n-propyl)heptyl group, a 3,4-dihydroxy-4-(n-propyl)heptyl group, a 3,5-dihydroxy-4-(n-propyl)heptyl group, a 4,5-dihydroxy-4-(n-propyl)heptyl group, a 4-hydroxy-5-methylhexyl group, a 5-hydroxy-5-methylhexyl group, a 6-hydroxy-5-methylhexyl group, a 4,5-dihydroxy-5-methylhexyl group, a 4,6-dihydroxy-5-methylhexyl group, a 5,6-dihydroxy-5-methylhexyl group, a 5-ethyl-4-hydroxyheptyl group, a 5-ethyl-5-hydroxyheptyl group, a 5-ethyl-6-hydroxyheptyl group, a 5-ethyl-4,5-dihydroxyheptyl group, a 5-ethyl-4,6-dihydroxyheptyl group, a 5-ethyl-5,6-dihydroxyheptyl group, a 4-hydroxy-5-(n-propyl)octyl group, a 5-hydroxy-5-(n-propyl)octyl group, a 6-hydroxy-5-(n-propyl)octyl group, a 4,5-dihydroxy-5-(n-propyl)octyl group, a 4,6-dihydroxy-5-(n-propyl)octyl group, a 5,6-dihydroxy-5-(n-propyl)octyl group, a 5-hydroxy-6-methylheptyl group, a 6-hydroxy-6-methylheptyl group, a 7-hydroxy-6-methylheptyl group, a 5,6-dihydroxy-6-methylheptyl group, a 5,7-dihydroxy-6-methylheptyl group, a 6,7-dihydroxy-6-methylheptyl group, a 6-ethyl-5-hydroxyoctyl group, a 6-ethyl-6-hydroxyoctyl group, a 6-ethyl-7-hydroxyoctyl group, a 6-ethyl-5,6-hydroxyoctyl group, a 6-ethyl-5,7-hydroxyoctyl group, a 6-ethyl-6,7- hydroxyoctyl group, a 5-hydroxy-6-(n-propyl)nonyl group, a 6-hydroxy-6-(n-propyl)nonyl group, a 7-hydroxy-6-(n-propyl)nonyl group, a 5,6-dihydroxy-6-(n-propyl)nonyl group, a 5,7-dihydroxy-6-(n-propyl)nonyl group and a 6,7-dihydroxy-6-(n-propyl)nonyl group; a 4-hydroxy-4-methyl-2-pentenyl group, a 5-hydroxy-4-methyl-2-pentenyl group, a 4,5-dihydroxy-4-methyl-2-pentenyl group, a 4-ethyl-4- hydroxy-2-hexenyl group, a 4-ethyl-5-hydroxy-2-hexenyl group, a 4-ethyl-4,5-dihydroxy-2-hexenyl group, a 4-hydroxy-4-(n-propyl)-2-heptenyl group, a 5-hydroxy-4-(n-propyl)-2-heptenyl group, a 4,5-dihydroxy-4-(n-propyl)-2-heptenyl group, a 5-hydroxy-5-methyl-3-hexenyl group, a 6-hydroxy-5-methyl-3-hexenyl group, a 5,6-dihydroxy-5-methyl-3-hexenyl group, a 5-ethyl-5-hydroxy-3-heptenyl group, a 5-ethyl-6-hydroxy-3-heptenyl group, a 5-ethyl-5,6-dihydroxy-3-heptenyl group, a 5-hydroxy-5-(n-propyl)-3-octenyl group, a 6-hydroxy-5-(n-propyl)-3-octenyl group, a 5,6-dihydroxy-5-(n-propyl)-3-octenyl group, a 4-hydroxy-5-methyl-2-hexenyl group, a 5-hydroxy-5-methyl-2-hexenyl group, a 6-hydroxy-5-methyl-2-hexenyl group, a 4,5-dihydroxy-5-methyl-2-hexenyl group, a 4,6-dihydroxy-5-methyl-2-hexenyl group, a 5,6-dihydroxy-5-methyl-2-hexenyl group, a 5-ethyl-4-hydroxy-2-heptenyl group, a 5-ethyl-5-hydroxy-2-heptenyl group, a 5-ethyl-6-hydroxy-2-heptenyl group, a 5-ethyl-4,5-dihydroxy-2-heptenyl group, a 5-ethyl-4,6-dihydroxy-2-heptenyl group, a 5-ethyl-5,6-dihydroxy-2-heptenyl group, a 4-hydroxy-5-(n-propyl)-2-octenyl group, a 5-hydroxy-5-(n-propyl)-2-octenyl group, a 6-hydroxy-5-(n-propyl)-2-octenyl group, a 4,5-dihydroxy-5-(n-propyl)-2-octenyl group, a 4,6-dihydroxy-5-(n-propyl)-2-octenyl group, a 5,6-dihydroxy-5-(n-propyl)-2-octenyl group, a 6-hydroxy-6-methyl-4-heptenyl group, a 7-hydroxy-6-methyl-4-heptenyl group, a 6,7-dihydroxy-6-methyl-4-heptenyl group, a 6-ethyl-6-hydroxy-4-octenyl group, a 6-ethyl-7-hydroxy-4-octenyl group, a 6-ethyl-6,7-dihydroxy-4-octenyl group, a 6-hydroxy-6-(n-propyl)-4-nonenyl group, a 7-hydroxy-6-(n-propyl)-4-nonenyl group, a 6,7-dihydroxy-6-(n-propyl)-4-nonenyl group, a 5-hydroxy-6-methyl-3-heptenyl group, a 6-hydroxy-6-methyl-3-heptenyl group, a 7-hydroxy-6-methyl-3-heptenyl group, a 5,6-dihydroxy-6-methyl-3-heptenyl group, a 5,7-dihydroxy-6-methyl-3-heptenyl group, a 6,7-dihydroxy-6-methyl-3-heptenyl group, a 6-ethyl-5-hydroxy-3-octenyl group, a 6-ethyl-6-hydroxy-3-octenyl group, a 6-ethyl-7-hydroxy-3-octenyl group, a 6-ethyl-5,6-dihydroxy-3-octenyl group, a 6-ethyl-5,7-dihydroxy-3-octenyl group, a 6-ethyl-6,7-dihydroxy-3-octenyl group, a 5-hydroxy-6-(n-propyl)-3-nonenyl group, a 6-hydroxy-6-(n-propyl)-3-nonenyl group, a 7-hydroxy-6-(n-propyl)-3-nonenyl group, a 5,6-dihydroxy-6-(n-propyl)-3-nonenyl group, a 5,7-dihydroxy-6-(n-propyl)-3-nonenyl group, a 6,7-dihydroxy-6-(n-propyl)-3-nonenyl group, a 5-hydroxy-6-methyl-2-heptenyl group, a 6-hydroxy-6-methyl-2-heptenyl group, a 7-hydroxy-6-methyl-2-heptenyl group, a 5,6-dihydroxy-6-methyl-2-heptenyl group, a 5,7-dihydroxy-6-methyl-2-heptenyl group, a 6,7-dihydroxy-6-methyl-2-heptenyl group, a 6-ethyl-5-hydroxy-2-octenyl group, a 6-ethyl-6-hydroxy-2-octenyl group, a 6-ethyl-7-hydroxy-2-octenyl group, a 6-ethyl-5,6-dihydroxy-2-octenyl group, a 6-ethyl-5,7-dihydroxy-2-octenyl group, a 6-ethyl-6,7-dihydroxy-2-octenyl group, a 5-hydroxy-6-(n-propyl)-2-nonenyl group, a 6-hydroxy-6-(n-propyl)-2-nonenyl group, a 7-hydroxy-6-(n-propyl)-2-nonenyl group, a 5,6-dihydroxy-6-(n-propyl)-2-nonenyl group, a 5,7-dihydroxy-6-(n-propyl)-2-nonenyl group, a 6,7-dihydroxy-6-(n-propyl)-2-nonenyl group, a 4-hydroxy-4-methyl-2-pentynyl group, a 5-hydroxy-4-methyl-2-pentynyl group, a 4,5-dihydroxy-4-methyl-2-pentynyl group, a 4-ethyl-4-hydroxy-2-hexynyl group, a 4-ethyl-5-hydroxy-2-hexynyl group, a 4-ethyl-4,5-dihydroxy-2-hexynyl group, a 4-hydroxy-4-(n-propyl)-2-heptynyl group, a 4-hydroxy-4-(n-propyl)-2-heptynyl group, a 5-hydroxy-4-(n-propyl)-2-heptynyl group, a 4,5-dihydroxy-4-(n-propyl)-2-heptynyl group, a 5-hydroxy-5-methyl-3-hexynyl group, a 6-hydroxy-5-methyl-3-hexynyl group, a 5,6-dihydroxy-5-methyl-3-hexynyl group, a 5-ethyl-5-hydroxy-3-heptynyl group, a 5-ethyl-6-hydroxy-3-heptynyl group, a 5-ethyl-5,6-dihydroxy-3-heptynyl group, a 5-hydroxy-5-(n-propyl)-3-octynyl group, a 6-hydroxy-5-(n-propyl)-3-octynyl group, a 5,6-dihydroxy- 5-(n-propyl)-3-octynyl group, a 4-hydroxy-5-methyl-2-hexynyl group, a 5-hydroxy-5-methyl-2-hexynyl group, a 6-hydroxy-5-methyl-2-hexynyl group, a 4,5-dihydroxy-5-methyl-2-hexynyl group, a 4,6-dihydroxy-5-methyl-2-hexynyl group, a 5,6-dihydroxy-5-methyl-2-hexynyl group, a 5-ethyl-4-hydroxy-2-heptynyl group, a 5-ethyl-5-hydroxy-2-heptynyl group, a 5-ethyl-6-hydroxy-2-heptynyl group, a 5-ethyl-4,5-dihydroxy-2-heptynyl group, a 5-ethyl-4,6-dihydroxy-2- heptynyl group, a 5-ethyl-5,6-dihydroxy-2-heptynyl group, a 4-hydroxy-5-(n-propyl)-2-octynyl group, a 5-hydroxy-5-(n- propyl)-2-octynyl group, a 6-hydroxy-5-(n-propyl)-2-octynyl group, a 4,5-dihydroxy-5-(n-propyl)-2-octynyl group, a 4,6-dihydroxy-5-(n-propyl)-2-octynyl group, a 5,6-dihydroxy-5-(n-propyl)-2-octynyl group, a 6-hydroxy-6-methyl-4-heptynyl group, a 7-hydroxy-6-methyl-4-heptynyl group, a 6,7-dihydroxy-6-methyl-4-heptynyl group, a 6-ethyl-6-hydroxy-4-octynyl group, a 6-ethyl-7-hydroxy-4-octynyl group, a 6-ethyl-6,7-dihydroxy-4-octynyl group, a 6-hydroxy-6-(n-propyl)-4-nonynyl group, a 7-hydroxy-6-(n-propyl)-4-nonynyl group, a 6,7-dihydroxy-6-(n-propyl)-4-nonynyl group, a 5-hydroxy-6-methyl-3-heptynyl group, a 6-hydroxy-6-methyl-3-heptynyl group, a 7-hydroxy-6-methyl-3-heptynyl group, a 5,6-dihydroxy-6-methyl-3-heptynyl group, a 5,7-dihydroxy-6-methyl-3-heptynyl group, a 6,7-dihydroxy-6-methyl-3-heptynyl group, a 6-ethyl-5-hydroxy-3-octynyl group, a 6-ethyl-6-hydroxy-3-octynyl group, a 6-ethyl-7-hydroxy-3-octynyl group, a 6-ethyl-5,6-dihydroxy-3-octynyl group, a 6-ethyl-5,7-dihydroxy-3-octynyl group, a 6-ethyl-6,7-dihydroxy-3-octynyl group, a 5-hydroxy-6-(n-propyl)-3-nonynyl group, a 6-hydroxy-6-(n-propyl)-3-nonynyl group, a 7-hydroxy-6-(n-propyl)-3-nonynyl group, a 5,6-dihydroxy-6-(n-propyl)-3-nonynyl group, a 5,7-dihydroxy-6-(n-propyl)-3-nonynyl group, a 6,7-dihydroxy-6-(n-propyl)-3-nonynyl group, a 5-hydroxy-6-methyl-2-heptynyl group, a 6-hydroxy-6-methyl-2-heptynyl group, a 7-hydroxy-6-methyl-2-heptynyl group, a 5,6-dihydroxy-6-methyl-2-heptynyl group, a 5,7-dihydroxy-6-methyl-2-heptynyl group, a 6,7-dihydroxy-6-methyl-2-heptynyl group, a 6-ethyl-5-hydroxy-2-octynyl group, a 6-ethyl-6-hydroxy-2-octynyl

group, a 6-ethyl-7-hydroxy-2-octynyl group, a 6-ethyl-5,6-dihydroxy-2-octynyl group, a 6-ethyl-5,7-dihydroxy-2-octynyl group, a 6-ethyl-6,7-dihydroxy-2-octynyl group, a 5-hydroxy-6-(n-propyl)-2-nonynyl group, a 6-hydroxy-6-(n-propyl)-2-nonynyl group, a 7-hydroxy-6-(n-propyl)-2-nonynyl group, a 5,6-dihydroxy-6-(n-propyl)-2-nonynyl group, a 5,7-dihydroxy-6-(n-propyl)-2-nonynyl group and a 6,7-dihydroxy-6-(n-propyl)-2-nonynyl group. Preferred examples are a 3-hydroxy-3-methylbutyl group, a 4-hydroxy-3-methylbutyl group, a 3,4-dihydroxy-3-methylbutyl group, a 3-ethyl-3-hydroxypentyl group, a 3-ethyl-4-hydroxypentyl group, a 3-ethyl-3,4-dihydroxypentyl group, a 4-hydroxy-4-methylpentyl group, a 5-hydroxy-4-methylpentyl group, a 4,5-dihydroxy-4-methylpentyl group, a 4-ethyl-4-hydroxyhexyl group, a 4-ethyl-5-hydroxyhexyl group, a 4-ethyl-4,5-dihydroxyhexyl group, a 4-hydroxy-4-methyl-2-pentenyl group, a 5-hydroxy-4-methyl-2-pentenyl group, a 4,5-dihydroxy-4-methyl-2-pentenyl group, a 4- ethyl-4-hydroxy-2-hexenyl group, a 4-ethyl-5-hydroxy-2-hexenyl group, a 4-ethyl-4,5-dihydroxy-2-hexenyl, a 4-hydroxy-4-methyl-2-pentynyl group, a 5-hydroxy-4-methyl-2-pentynyl group, a 4,5-dihydroxy-4-methyl-2-pentynyl group, a 4-ethyl-4-hydroxy-2-hexynyl group, a 4-ethyl-5-hydroxy-2-hexynyl group and a 4-ethyl-4,5-dihydroxy-2-hexynyl group.

Herein, the alkyl group generally refers to a straight chain or branched chain alkyl group having 1 to 15 carbon atoms, preferably 1 to 8 carbon atoms. The aryl group generally refers to an aryl group having 6 to 20 carbon atoms, preferably 6 to 14 carbon atoms. The alkoxy group generally refers to a straight chain or branched chain alkoxy group having 1 to 15 carbon atoms, preferably 1 to 8 carbon atoms.

The protecting group includes, for example, an acyl group, a substituted silyl group and a substituted alkyl group, and is preferably an acyl group or a substituted silyl group.

The acyl group refers to a substituted carbonyl group, in which the substituent for the carbonyl group refers to a hydrogen atom, an optionally substituted lower alkyl group, an optionally substituted aryl group, an optionally substituted lower alkyloxy group, an optionally substituted aryloxy group or an optionally substituted aralkyloxy group. The acyl group preferably refers to a formyl group, a lower alkylcarbonyl group, a phenylcarbonyl group optionally having a substituent, a lower alkyloxycarbonyl group or an optionally substituted phenylalkyloxycarbonyl group, and more preferably a formyl group, an acetyl group, a propionyl group, a butyryl group, a pivaloyl group, a benzoyl group, a methoxycarbonyl group, an ethoxycarbonyl, a t-butoxycarbonyl group or a benzyloxycarbonyl group.

The substituted silyl group refers to a silyl group substituted by a lower alkyl group optionally having one or more substituents or by an optionally substituted aryl group. Preferably, the substituted silyl group refers to a trisubstituted silyl group. Preferred examples of the substituted silyl group are a trimethylsilyl group, a triethylsilyl group, a triisopropylsilyl group, a t-butyldiphenylsilyl group and a t-butyldimethylsilyl group.

The substituted alkyl group refers to an alkyl group substituted by one or more substituents. Here, preferred examples of the substituent are an optionally substituted alkyloxy group and an optionally substituted aryl group, especially an optionally substituted alkyloxy group. Examples of the substituted alkyl group substituted by the optionally substituted alkyloxy group, such as an alkyloxy group, are a methoxymethyl group, a 2-methoxyethoxymethyl group and a tetrahydropyran-2-yl group. Examples of the substituent include a halogen atom, a cyano group, a nitro group, an amino group, a hydroxyl group, an alkyl group, an alkyloxy group, an acyloxy group and a sulfonyl group.

Of the compounds represented by the general formula (1) of the present invention, compounds having a sulfur atom as X can be prepared by, for example, the method described in Japanese Unexamined Patent Publication No. 1998-231284.

As the compounds to become the active ingredient of the drug for treatment of osteoporosis according to the present invention, there can be preferably used 1,3-dihydroxy-20-(3-hydroxy-3-methylbutylthio)-9,10-secopregna-5,7,10(19),16-tetraene, 1α,3β-dihydroxy-20(S)-(3-hydroxy-3-methylbutylthio)-9,10-secopregna-5,7,10(19),16-tetraene, 1α,3β-dihydroxy-20(R)-(3-hydroxy-3-methylbutylthio)-9,10-secopregna-5,7,10(19),16-tetraene, 1α,3β-dihydroxy-20(R)-((E)-4-hydroxy-4-methyl-2-pentenylthio)-9,10-secopregna-5,7,10(19),16-tetraene, 1α,3β-dihydroxy-20(R)-((E)-4-ethyl-4-hydroxy-2-hexenylthio)-9,10-secopregna-5,7,10(19),16-tetraene, 1α,3β-dihydroxy-20(S)-(2-hydroxy-2-methylpropylthio)-9,10-secopregna-5,7,10(19),16-tetraene, 1α,3β-dihydroxy-20(R)-(2-hydroxy-2-methylpropylthio)-9,10-secopregna-5,7,10(19),16-tetraene, 1α,3β-dihydroxy-20(S)-{2(S)-hydroxy-3-methylbutyloxy}-9,10-secopregna-5,7,10(19),16-tetraene, 1α,3β-dihydroxy-20(S)-{2(R)-hydroxy-3-methylbutyloxy}-9,10-secopregna-5,7,10(19),16-tetraene, 1α,3β-dihydroxy-20(S)-(2-ethyl-2-hydroxybutylthio)-9,10-secopregna-5,7,10(19),16-tetraene, 1α,3β-dihydroxy-20(R)-(2-ethyl-2-hydroxybutylthio)-9,10-secopregna-5,7,10(19),16-tetraene, 1α,3β-dihydroxy-20(R)-(4-ethyl-4-hydroxy-2-hexynyloxy)-9,10-secopregna-5,7,10(19),16-tetraene and 1α,3β-dihydroxy-20(R)-(3-ethyl-2(S)-hydroxypentylthio}-9,10-secopregna-5,7,10(19),16-tetraene.

In the screening method of the present invention, the VDRE-mediated activity of the test compound to promote transcription is measured. VDRE is a kind of regulatory sequence on DNA. The expression of a gene located downstream of VDRE is activated when a vitamin D receptor bound to a ligand (e.g. a vitamin D derivative) binds to and acts on VDRE. For example, hypercalcemia, an adverse reaction of vitamin D, is believed to result via VDRE. The DNA base sequence of VDRE may differ according to the species of organism and the type of target genes for regulation even among the same biological species. Any VDRE sequence may be used in the present invention. In the Examples to be offered later on, screening was carried out by means of a reporter gene assay system using the VDRE sequence

of a mouse osteopontin gene, but this is only one example of the VDRE sequence. Other examples of the VDRE sequence are rat osteocalcin gene, human osteocalcin gene, rat calbindin-$D_{9K}$ gene, human calbindin-$D_{9K}$ gene, mouse calbindin-$D_{28K}$ gene, rat 24-hydroxylase gene, human 24-hydroxylase gene and chicken integrin β3 gene. They can also be used in the method of the present invention.

In the screening method of the present invention, the inhibitory action of the test compound against the activity of a transcription factor is measured. The transcription factor generally refers to a proteinous factor necessary for a transcription reaction from DNA into RNA in the nucleus. In the present invention, the transcription factor refers, particularly, to a factor which recognizes a particular base sequence existing upstream of a particular gene and binds to the sequence, thereby controlling the expression of the gene. The transcription factor used in the screening method of the present invention is not restricted in type, as long as its activity is inhibited by a vitamin D receptor. Examples of the transcription factor are AP-1 complex, NF-κB family, Sp1 family, Oct family, ATF/CREB family, NF-AT family, STAT family and Egr family. Preferred examples are AP-1 complex and NF-κB family used in the examples (to be offered later on) of the specification.

AP-1 (activator protein-1) complex is a transcription factor composed of a homodimer of Jun family or a heterodimer of Fos family and Jun family. AP-1 complex binds to a TRE sequence which is an AP-1 binding site. As the Jun family, c-Jun, JunB and JunD have been identified. As the Fos family, c-Fos, FosB, Fra-1 and Fra-2 have been identified.

As genes having the AP-1 binding site, cytokines such as interleukin-1, interleukin-2, interleukin-5, GM-CSF and TNF-α, adhesion molecules such as ICAM-1 and enzymes such as collagenase are known. A reporter gene assay using the AP-1 binding site of any of these genes is also usable. The DNA base sequence of the AP-1 binding site may differ according to the species of organism and the type of target genes for regulation even among the same biological species. Any sequence of the AP-1 binding site may be used in the present invention.

NF-κB (nuclear factor-κB) family is a transcription factor composed of a dimmer of Rel family and binding to a κB sequence, an NF-κB binding site. As the Rel family, NF-κBp50, NF-κBp52, NF-κBp65 (RelA), c-Rel and RelB have been identified.

As genes having the NF-κB binding site, cytokines such as interleukin-1, interleukin-2, interleukin-6, interleukin-8 and TNF-α and adhesion molecules such as ICAM-1 and VCAM-1 are known. A reporter gene assay using the NF-κB binding site of any of these genes is also usable. The DNA base sequence of the NF-κB binding site may differ according to the species of organism and the type of target genes for regulation even among the same biological species. Any sequence of the NF-κB binding site may be used in the present invention.

A characteristic feature of the screening method of the present invention is its ability to measure whether or not a vitamin D receptor shows any inhibitory action against the activity of a transcription factor. Depending on the transcription factor used, however, the vitamin D receptor may show an activating action (activity promoting action), rather than an inhibitory action against the activity. When such a transcription factor is used, therefore, the activity promoting action, rather than the activity inhibiting action, is to be measured. This measurement is also within the scope of the present invention.

Of the above-described transcription factors, AP-1 complex, for example, is known to bind to the AP-1 binding site to activate the expression of a gene located downstream therefrom, thereby causing bone destruction. Thus, if the activation of AP-1 complex can be inhibited by a vitamin D receptor bound to a vitamin D derivative, it is expected to inhibit the expression of the above-mentioned gene which causes bone destruction. If an activation factor for expression of a gene potentially involved in the onset or progression of some disease (e.g. bone destruction or inflammation) has been identified and the action of the activation factor has been found to be inhibitable by a vitamin D receptor, it becomes possible to develop an advantageous drug with decreased adverse effects by selecting a compound which has higher selectivity towards an inhibitory activity against the activation factor than VDRE-mediated transcription promoting activity (i.e., a compound with a stronger inhibitory action against activity of the transcription factor, relative to VDRE-mediated transcription promoting activity) by the screening method of the present invention. Hence, the transcription factor in the present invention is preferably one relating to the onset or progression of a disease such as bone destruction or inflammation.

The measurement of VDRE-mediated transcription promoting activity and inhibitory action against the activity of the transcription factor can be accomplished by any method known in the art and the method is not restrictive. Preferably, the reporter gene assay method is named. The reporter gene assay method refers to a method which comprises, for example, constructing each of a vector composed of a VDRE sequence, a promoter sequence and a preferred reporter gene connected together in this order, and an expression vector for a vitamin D receptor; transfecting these vectors simultaneously into an appropriate host; adding a test substance and culturing the transfected cells; and measuring the amount of the reporter gene expressed in the cells to evaluate the VDRE-mediated transcription promoting activity of the test substance.

The type of the host transfected with the vector containing the reporter gene is not restricted, and any person skilled in the art can select, as desired, any preferred host in conventional use in the field. Nonrestrictive examples of

the host cell are cells of human origin, such as Jurkat cells, HeLa cells, HepG2, CaCO-2, SaOS and K562; cells of monkey origin, such as CV-1, COS-1 and COS-7; cells of mouse origin, such as NIH3T3, L929, F9 and MC-3T3-E1; cells of rat origin, such as PC-12 and ROS17/2.8; and cells of hamster origin, such as CHO-K1 and BHK-21.

The method of transfection of the vector into the host is not restricted and can be selected, as desired, by a person skilled in the art from among the methods commonly used in the field. Nonrestrictive examples of the transfection method are calcium phosphate transfection, DEAE-dextran transfection, electroporation and lipofection.

The reporter gene is a marker gene integrated into DNA to investigate the transcription activity of the promoter or enhancer. It is not restricted and may be any gene the amount of which is expressed can be measured. Generally, a gene which is easy to detect and quantitatively measurable is preferred. For example, a gene for an enzyme catalyzing a color development or luminescence reaction is preferred. In this case, the amount of the enzyme expressed, i.e., transcription promoting activity, can be evaluated by adding a substrate for a color development reaction to the cell lysate and measuring the degree of color development produced. Alternatively, the amount of expression of the reporter gene can be measured by quantitatively measuring protein by ELISA or the like using an antibody.

Examples of the reporter gene are CAT (chloramphenicol acetyltransferase) gene, Luc (luciferase) gene, β-Gal (β-galactosidase) gene, hGH (secretory human growth factor) gene, SEAP (human secretory alkaline phosphatase) gene, GFP (green fluorescent protein) gene and GUS (β-glucuronidase) gene.

In the CAT assay using CAT gene as the reporter gene, a reporter gene vector, in which the base sequence of a regulatory region the transcription activity of which should be measured has been joined to an upstream region of CAT gene, is introduced into cells by transfection or the like to cause transient expression. Generally, a cell extract is prepared in 12 to 72 hours and acetyl-CoA and chloramphenicol are added thereto for reaction.' The acetylated chloramphenicol is separated and identified by using thin layer chromatography or the like. That is, according to the CAT assay, the transcription activity of the gene is measured as the enzyme activity of acetylating chloramphenicol.

Luciferase is a generic name for luminescent enzymes which catalyze a light-emitting reaction expressed by the formula:

$$\text{Luciferin} + O_2 \rightarrow \text{Oxyluciferin} + \text{light}$$

The use of luciferase as a reporter gene makes it possible to cause a light emitting reaction to the cell extract given the substrate and oxygen and to evaluate the amount of expression of luciferase by measuring the degree of light emission.

The above-described reporter gene assay can be performed by conventional methods known among people skilled in the art which are described, for example, in S.K. Nordeen, Biotechniques, Vol. 6, pp. 454-456, (1988). Alternatively, the reporter gene assay method can be performed readily and conveniently with the use of a reporter gene assay reagent commercially available from Boehringer Mannheim Biochemicals, Clontech Laboratories Inc. or Promega Corporation in accordance with a manual attached thereto.

Alternative methods for evaluating the transcription promoting activity or the inhibitory action on the activity of the transcription factor include, for example, gel shift assay, run-on assay and run-off assay.

The gel shift assay is an easy, convenient and effective method for investigating a binding factor for DNA. Its principle is as follows: After DNA and a DNA binding factor are reacted, electrophoresis is performed using non-denatured polyacrylamide gel. The mobility of the protein-bound DNA is lower than the mobility of the protein-unbound DNA. Thus, the binding factor is detected using labeled DNA.

The run-on assay is also one of methods for investigating the transcription activity of a particular gene. Using nuclei isolated from cells, transcription is performed in vitro. In this case, new synthesis from the initiation point for transcription does not occur and only a synthesis reaction involving elongation of RNA is observed as a continuation of an RNA strand in which transcription has already started in the cellular state. Thus, the transcription product is labeled with a radioisotope in a system using the isolated nucleus and the gene to be investigated is selectively detected from the labeled RNA with the use of a probe. Based on this method, the transcription activity at the time of isolation of the nucleus of the particular gene is measured. In this manner, changes in the transcription activity over time and the relative amounts of transcription can be subjected to comparative study.

The run-off assay is also one of in vitro transcription methods, which linearizes template DNA to synthesize a constant length of a transcript. The template DNA is one cleaved by a restriction enzyme at a site several hundred bases downstream of the initiation point of transcription. When the linear template DNA is used for in vitro transcription, an RNA polymerase is dissociated from the template DNA at the cleavage site to terminate the transcription reaction. Thus, a constant length of RNA is formed. On this occasion, labeled ribonucleotides are incorporated during the reaction to label RNA. After electrophoresis, an autoradiogram is prepared and the amount of RNA is measured.

In the screening method of the present invention, a compound is selected which is more potent in inhibitory action against the activity of the transcription factor relative to its VDRE-mediated transcription promoting activity. This

is based on the prediction that useful physiological activities, such as a bone mass increasing action, occur in a situation in which there is no or minimal blood calcium increasing action, i.e. an adverse reaction of a vitamin D derivative. This is because the inhibitory action against the activity of the transcription factor appears more strongly than the VDRE-mediated transcription promoting activity. Hence, a degree of selectivity towards an inhibitory action against the activity of transcription factor, which is set for screening can be selected, as desired, depending on the uses of the compound to be selected, and should not be restricted to a particular value.

In a preferred mode of the present screening method, a standard substance can be used as a reference for evaluation of selectivity towards an inhibitory against the activity of a transcription factor. The type of standard substance is not restricted and any substance can be used as necessary. For example, 1$\alpha$,25-dihydroxyvitamin $D_3$ may be used as a standard substance, and a compound having higher selectivity towards an inhibitory action against the activity of a transcription factor than that of the standard substance can be chosen. By so doing, an advantageous compound with few adverse reactions can be searched for.

Particularly when the VDRE-mediated transcription promoting activity or the inhibitory action against the activity of the transcription factor is measured by the screening method of the present invention, the EC50 value or IC50 value of each test substance is generally measured, whereby each of the test substances can be evaluated for VDRE-mediated transcription promoting activity or inhibitory action against the activity of the transcription factor. Selectivity towards an inhibitory action against the activity of the transcription factor is evaluated based on a value expressed by the following equation:

[a relative value of the inhibitory action of the

test compound on the activity of the transcription factor

as compared with that of the standard substance (e.g.,

1$\alpha$,25-dihydroxyvitamin $D_3$)]/[a relative value of the VDRE-mediated

transcription promoting activity of the test

compound as compared with that of the standard substance

(e.g., 1$\alpha$,25-dihydroxyvitamin $D_3$)]

The test compound having the above value generally higher than 1, more preferably 10 or more, can be evaluated as a compound having higher selectivity towards the inhibitory action against activity of the transcription factor than does the standard substance (e.g., 1$\alpha$,25-dihydroxyvitamin $D_3$).

According to the present invention, there is provided a novel compound selected by the screening method of the present invention and having an affinity for a vitamin D receptor. Such a compound is useful as a drug for treatment of a disease the onset or progression of which the activation of a transcription factor has a role.

Examples of diseases the onset or progression of which the activation of a transcription factor is involved include metabolic bone diseases (e.g., osteoporosis, renal osteodystrophy), various inflammations (e.g., rheumatoid arthritis, osteoarthritis, asthma, arteriosclerosis, organ transplantation), various tumors (e.g., colon cancer, breast cancer, ATL), diabetes and myocardial infarction.

When the compound selected by the screening method of the present invention is used as a therapeutic drug for these diseases, its dosage form is not restricted. As desired, the compound can assume any appropriate form, such as a tablet, capsule, dispersion, solution, suspension or emulsion.

As for the route of administration, the compound may be administered orally or parenterally. In parenteral administration, any administration route, such as intravenous, intradermal, subcutaneous, intramuscular, intraperitoneal or external use, can be employed.

The dose is not restricted and can be set, as desired, according to body weight, sex, severity of disease, etc. of a patient. Generally, the dose is about 0.001 μg/kg/day to 1,000 μg/kg/day, preferably about 0.01 μg/kg/day to 100 μg/kg/day, more preferably about 0.1 μg/kg/day to 10 μg/kg/day.

The frequency of administration is not restricted, either. The above dose may be administered once daily or as up to several divided doses. The duration of treatment may be several days to several weeks or several weeks to several months.

According to the present invention, moreover, there is provided a kit for performing the screening method of the present invention, which includes a vector for evaluating the inhibitory action on activity of a transcription factor,

the vector containing a reporter gene; a vector for evaluating VDRE-mediated transcription promoting activity, the vector containing a reporter gene; if desired, a vector for expression of a vitamin D receptor; and a reagent for detecting a product of the reporter gene.

Using such a kit, host cells cultured under suitable conditions are transfected with either the vector for evaluating inhibitory activity against a transcription factor or the vector for evaluating VDRE-mediated transcription promoting activity and at the same time, transfected with the vitamin D receptor expression vector. Then, the test compound is added to the system and the cells are cultured as desired. Then, the cell lysate is recovered and the amount of expression of the reporter gene is evaluated by use of the reagent for detecting the product of the reporter gene. In this manner screening of the test substances can be performed readily. When cells highly expressive of a vitamin D receptor are used as the host cells, transfection with the vitamin D receptor expression vector is not necessarily required.

The entire description of Japanese Patent Application No. 1999-040090, the application on which the priority claim of the present application is based, is incorporated herein by reference in its entirety.

The present invention will be described more specifically by the following Examples, but the present invention is not restricted by these examples.

Example 1: Screening system

(A) Evaluation of inhibitory activity against AP-1 complex and NF-κB family by use of reporter gene assay system

(1) Reporter gene vector having an AP-1 binding sequence or an NF-κB binding sequence

A basic vector was constructed by inserting a promoter region (-72/+47) of a human IL-2 gene into a cassette vector of luciferase (pXP2; described in S.K. Nordeen, Biotechniques, Vol. 6, pp. 454-456, (1988)). For a reporter gene vector having an AP-1 binding sequence, five AP-1 binding sequences (ATGAGTCAG) of a human collagenase (MMP-1) gene were inserted tandem into the basic vector. For a reporter gene vector having an NF-κB binding sequence, four NF-κB binding sequences
(CAGAGGGGACTTTTCCGAGA) of a human immunoglobulin light chain κ gene were inserted tandem into the basic vector. The resulting products were used as the respective reporter gene vectors.

(2) Rat vitamin D receptor expression vector

The rat vitamin D receptor expression vector used was a product obtained by inserting cDNA of a rat vitamin D receptor (described in James K. Burmester et al., Proc. Natl. Acad. Sci. USA, Vol. 85, pp. 9499-9502, (1998)) into the pSG5 (STRATAGENE).

(3) Reporter gene assay using the reporter gene vector having the AP-1 binding sequence or NF-κB binding sequence

Jurkat cells were cultured in RPMI-1640 (GIBCO BRL) containing 10% FBS and antibiotics. The culture was started at a density of 5 x $10^4$ cells/mL and the cells were subcultured at intervals of 3 or 4 days to a density of 5 x $10^4$ cells/mL. The conditions for culture were 37°C and 5% $CO_2$. For transfection with the reporter gene vector, the solution A (5 µL LIPOFECT AMINE (GIBCO BRL), 0.5 mL OPTI-MEMI (GIBCO BRL)) and the solution B (0.5 mL OPTI-MEMI containing 1 µg each of the reporter gene vector and the rat vitamin D receptor expression vector) were prepared for 3 to 5 x $10^6$ Jurkat cells. Both of the solutions were mixed and then allowed to stand for 30 minute at room temperature. During this period, the cells were washed twice with serum-free medium. Thirty minutes later, the cells were suspended in the mixed solution and treated for 5 to 6 hours at 37°C in 5% $CO_2$. Then, RPMI-1640 containing 10% FBS and antibiotics was added so that the cell density would be 1 x $10^6$ cells/mL, whereafter the system was cultured overnight. On the following day, the cells were seeded into a 96-well black plate (Nunc.) at a density of 2 x $10^5$ cells/200 µL/well. PMA (phorbol 12-myristate 13-acetate) and ionomycin (SIGMA) were added as stimulants in amounts of 10 ng/mL and 1 µg/mL, respectively (10 µL of a diluted (1/20) solution of each of them was added). Further, vitamin D derivatives prepared at a one twentieth concentration were each added in an amount of 10 µL (the vitamin D derivatives were each diluted with a vehicle (RPMI-1640 containing 2% ethanol, 10% FBS and antibiotics) to a one twentieth concentration; for the control group, the vehicle was added in an amount of 10 µl/well).

The vitamin D derivatives tested were 28 types of compounds having the following structures (i.e., Compound No. 1 to Compound No. 28). As the standard substance, 1α,25-dihydroxyvitamin $D_3$ was used.

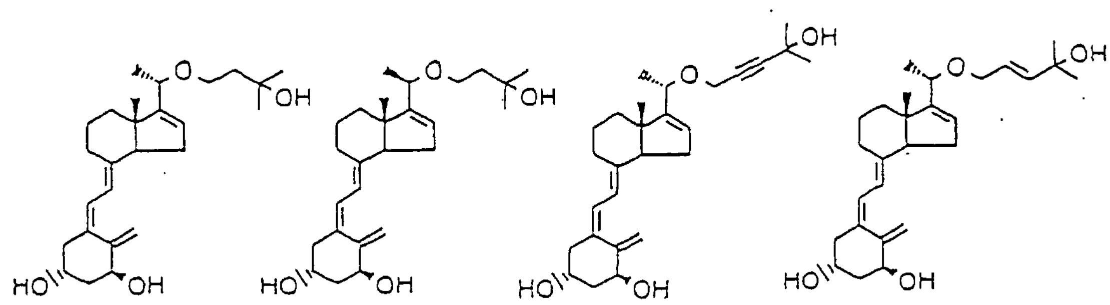

1 2 3 4

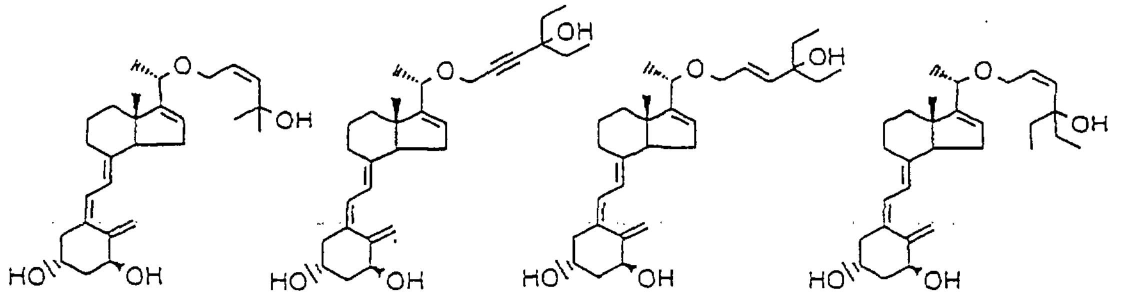

5 6 7 8

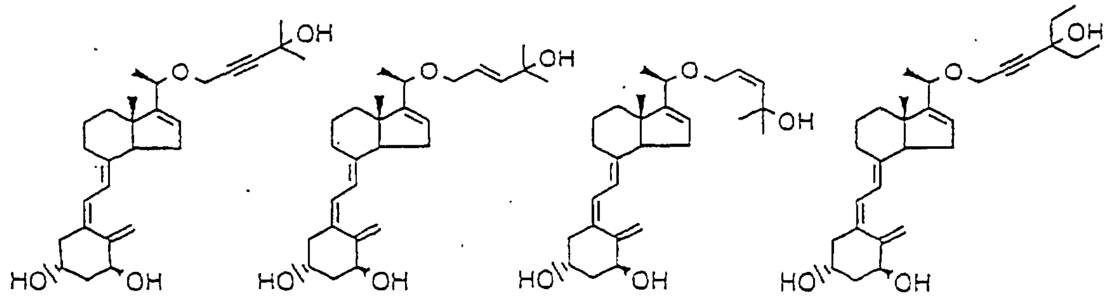

9 10 11 12

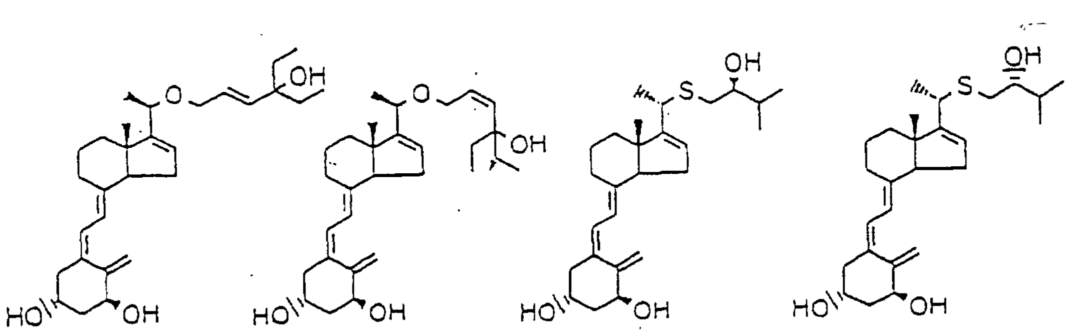

13 14 15 16

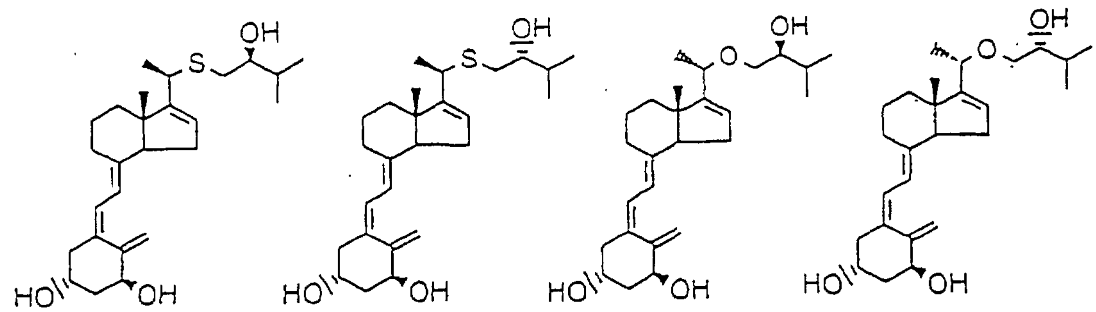

17 18 19 20

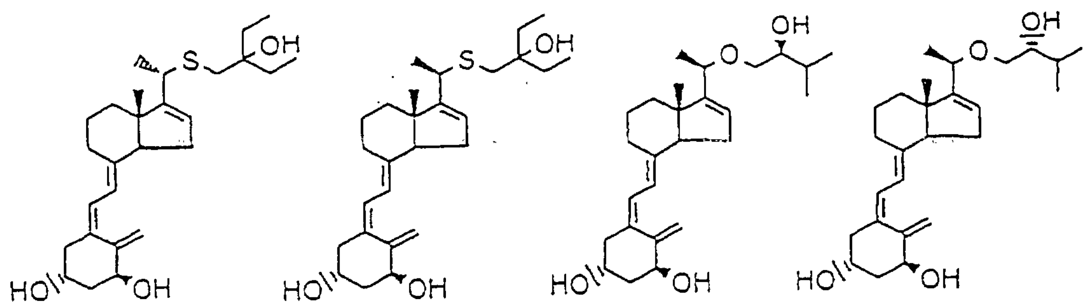

21 22 23 24

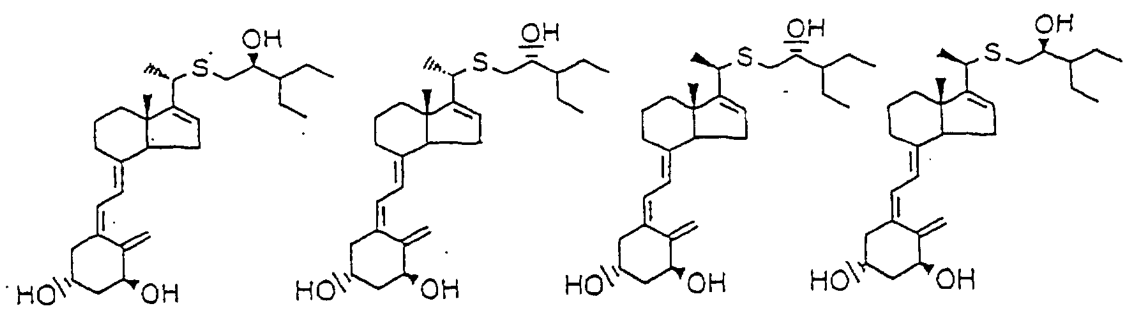

25 26 27 28

After 8 to 10 hours of culture, the plate was centrifuged (1,500 rpm, 15 min), and the culture supernatant was removed. The cells were lysed with 20 μL/well of Reporter Lysis Buffer (Promega), and the total amount of the lysate was used in a luciferase assay. In the luciferase assay, 100 μL of Luciferase Assay Reagent (Promega) was added to the cell lysate, and the fluorescence intensity over 5 seconds was measured with Luminoscan RS (Labsystems).

(4) Evaluation of inhibitory activity against AP-1 complex and NF-κB family (calculation of IC37.5 value)

Based on the measured values of the luciferase assay, the IC37.5 value of each vitamin D derivative was calculated to evaluate the inhibitory activity against AP-1 complex and NF-κB family. The measured value of the control obtained without the addition of the derivative was taken as the AP-1 complex or NF-κB family transcription activity of 100%, and the dose of each derivative that produced an inhibition of 37.5% was calculated as the IC37.5 value.

The IC37.5 values obtained are expressed as the percentages of the values of 1α,25-dihydroxyvitamin $D_3$, and the results are shown in Table 1. In the table, "-" represents that the test was not conducted.

Table 1

| Compound | Inhibitory activity against AP-1 complex | Inhibitory activity against NF-κB family |
|---|---|---|
| 1α,25-dihydroxyvitamin $D_3$ | 100.00 | 100.00 |
| 1 | 17.59 | - |
| 2 | 18.79 | - |
| 3 | 13.91 | 46.54 |
| 4 | 24.09 | 13.84 |
| 5 | 3.85 | 2.90 |
| 6 | 16.67 | 2.50 |
| 7 | 2.30 | 3.94 |
| 8 | 10.12 | 3.55 |
| 9 | 15.82 | 38.80 |
| 10 | 1262.83 | 520.18 |
| 11 | 6.29 | 4.78 |
| 12 | 0.95 | 12.45 |
| 13 | 4.15 | 12.32 |
| 14 | 2.19 | 10.21 |
| 15 | 4.29 | 3.86 |
| 16 | 3.92 | 10.88 |
| 17 | 31.22 | 63.51 |
| 18 | 6.60 | 9.63 |
| 19 | 1.88 | 19.63 |
| 20 | 14.95 | 29.37 |
| 21 | 2.41 | 4.21 |
| 22 | 3.74 | 5.19 |
| 23 | 171.89 | 157.78 |
| 24 | 20.55 | 36.87 |
| 25 | 6.37 | 1.90 |
| 26 | 22.24 | 6.42 |
| 27 | 41.80 | 24.74 |
| 28 | 20.98 | 26.13 |

(B) Evaluation of VDRE-mediated transcription promoting activity by use of reporter gene assay system

(1) Reporter gene vector having VDRE

The reporter gene vector having VDRE used was a product produced by integrating VDRE (GGTTCACGAGGTTCA) of a mouse osteopontin gene into a cassette vector (pGCAT; Kato S. et al., Cell (1992)68, 731-742) containing a promoter region (-109/+10) of rabbit β-globin and CAT (chloramphenicol acetyltransferase). As an expression vector for β-galactosidase used for correction of transfection efficiency, pCH110 (Pharmacia) was used. As a carrier vector, BSM (BluescribeM13+: STRATAGENE) was used.

(2) Rat vitamin D receptor expression vector

The rat vitamin D receptor expression vector used was a product obtained by integrating cDNA of a rat vitamin D receptor into pSG5 (STRATAGENE).

(3) Reporter gene assay using the reporter gene vector having VDRE

COS-1 cells were cultured in a DMEM medium containing 5% FBS and 10 nM insulin, and cultures were subcultured at intervals of 3 or 4 days at a density of 1,500 to 3,000 cells/$cm^2$. The conditions for culture were 37°C and 5% $CO_2$. For cultivation of COS-1 cells during reporter gene assay, a phenol red-free DMEM medium containing 5% DCC-FBS (charcoal-treated FBS) and 10 nM insulin was used. COS-1 cells were seeded into a 6-well plate at a density of 5 to 8 x $10^4$ cells/1.75 mL/well. After overnight culture, the cells were transfected with the reporter gene vector, etc. by the calcium phosphate method.

Specifically, transfection was performed in the following manner: 3.33 μg DNA/35 μL TE solution (TE containing 0.33 μg reporter gene vector, 0.08 μg rat vitamin D receptor expression vector, 0.5 μg pCH110 and 2.42 μg BSM; TE comprising 10 mM Tris-HCl (pH 7.5) and 1 mM EDTA) was prepared for each well. To the TE solution, 5 μL of 2M $CaCl_2$ was added. Further, 40 μL of HBS (280 mM NaCl, 50 mM HEPES, 1.5 mM $Na_2HPO_4$) was added with gentle mixing and the mixture was allowed to stand for 1 hour'at room temperature. Then, 80 μL of the resulting solution was added to each well. Also, 17.5 μL of each of the vitamin D derivatives prepared at a hundredth concentration was added (the vitamin D derivative was diluted with a vehicle (DMEM containing 10% ethanol) to a hundredth concentration; for the control group, the vehicle was added).

After overnight culture, each well was washed with 1 mL/well of a phenol red-free DMEM medium. Then, the vitamin D derivative was added again together with 1.75 mL/well of a phenol red-free DMEM medium containing 5% DCC-FBS and 10 nM insulin and the mixture was further cultured overnight. Then, each well was washed with 2 mL of PBS and the cells were scraped off in 1 mL of PBS by means of a cell scraper and recovered into a tube. After centrifugation, the cells were suspended in 150 μL of 0.25M Tris-HCl (pH 7.8) and a cell lysate was prepared by the freezing and thawing method. Using this cell lysate, β-galactosidase assay was performed for correction of the transfection efficiency. Then, the amount of CAT expressed was measured using a CAT ELISA kit (Boehringer Mannheim).

(4) β-galactosidase assay

The cell lysate (10 μL), 175 μL of Buffer Z (60 mM $Na_2HPO_4$, 40 mM $NaH_2PO_4$, 10 mM KCl, 1 mM $MgSO_4$) and 35 μL of 4 mg/mL ONPG (o-nitrophenyl-β-D-galactopyranoside; SIGMA) were mixed in a 96-well plate (Nunc-Immuno plate Maxi Sorp Surface) and the system was incubated for 1 hour at 37°C. Then, 100 μL of 1M $Na_2CO_3$ was added to terminate the reaction and A415 was measured with a plate reader. Based on the measured values, the number of units of the cell lysate was calculated from the following equation and the cell lysate in an amount corresponding to 15 units was used in CAT ELISA measurement.

$$\text{Unit}/\mu\text{L} = (\text{A415} \times 100)/(\text{amount of cell lysate} \times 2 \times \text{reaction time}) = \text{A415} \times 5$$

(5) Calculation of EC50 value

Based on the measured values of CAT ELISA, the EC50 value of each derivative was calculated to evaluate the VDRE-mediated action. The maximum amount of CAT whose expression was induced by 1α,25-dihydroxyvitamin $D_3$ was taken as the reference value of 100% and the dose of each derivative that induced a CAT expression of 50% was calculated as the EC50 value.

The results are shown in Table 2 as relative values, with the EC50 value of 1α,25-dihydroxyvitamin $D_3$ as 100%.

Table 2

| Compound | VDRE-mediated transcription promoting activity |
|---|---|
| 1α,25-dihydroxyvitamin $D_3$ | 100.00 |
| 1 | 82.17 |

Table 2 (continued)

| Compound | VDRE-mediated transcription promoting activity |
|---|---|
| 1α,25-dihydroxyvitamin $D_3$ | 100.00 |
| 2 | 25.58 |
| 3 | 13.60 |
| 4 | 12.70 |
| 5 | 12.60 |
| 6 | 14.30 |
| 7 | 13.00 |
| 8 | 23.80 |
| 9 | 13.30 |
| 10 | 23.80 |
| 11 | 7.20 |
| 12 | 140.27 |
| 13 | 37.90 |
| 14 | 22.20 |
| 15 | 101.80 |
| 16 | 82.00 |
| 17 | 924.75 |
| 18 | 127.30 |
| 19 | 466.85 |
| 20 | 607.10 |
| 21 | 136.35 |
| 22 | 1032.20 |
| 23 | 234.60 |
| 24 | 207.70 |
| 25 | 15.30 |
| 26 | 19.20 |
| 27 | 334.50 |
| 28 | 1517.40 |

(C) Results of screening VDRE-mediated transcription promoting activity and inhibitory activity against AP-1 complex or NF-κB family

The various vitamin D derivatives described above were measured for the VDRE-mediated transcription promoting activity and the inhibitory activity against AP-1 complex or NF-κB family and the disparity between both activities of the respective vitamin D derivatives was evaluated. Compared with 1α,25-dihydroxyvitamin $D_3$, a plurality of the derivatives were found to show excellent disparity between both activities.

The results obtained are shown in FIGS. 1 and 2. In FIG. 1, the vertical axis shows the VDRE-mediated transcription promoting activity as relative values with the EC50 value of 1α,25-dihydroxyvitamin $D_3$ as 100%, while the horizontal axis shows the inhibitory activity against AP-1 complex as relative values with the IC37.5 value of 1α, 25-dihydroxyvitamin $D_3$ as 100%.

In FIG. 2, the vertical axis shows the VDRE-mediated transcription promoting activity as relative values with the EC50 value of 1α,25-dihydroxyvitamin $D_3$ as 100%, while the horizontal axis shows the inhibitory activity against

NF-κB family as relative values with the IC37.5 value of 1α,25-dihydroxyvitamin $D_3$ as 100%.

The numbers in FIGS. 1 and 2 represent the compound numbers (Nos.).

The best derivative in terms of the disparity between both activities was the derivative designated as Compound No. 22 (i.e., 1α,3β-dihydroxy-20(R)-(2-ethyl-2-hydroxybutylthio)-9,10-secopregna-5,7,10(19),16-tetraene). Its inhibitory,activity against AP-1 complex or NF-κB family was about 25 to 35 times as strong as 1α,25-dihydroxyvitamin $D_3$ and its VDRE-mediated transcription promoting activity was about 1/10 of that of 1α,25-dihydroxyvitamin $D_3$.

The above results confirmed the possibility for separating the VDRE-mediated transcription promoting activity of vitamin D derivatives and their inhibitory activity against AP-1 complex or NF-κB family.

Example 2: Effect of Compound No. 22 and 1α,25-dihydroxyvitamin $D_3$ on bone mass decrease in osteoporosis model rats (subcutaneous treatment)

(A) Experimental method

7- to 9-week-old W-I female rats (Imamichi Institute for Animal Reproduction) were subject to ovariectomy. From the following day onward, la,25-dihydroxyvitamin $D_3$ or Compound No. 22 was subcutaneously administered 5 times weekly for 6 weeks in a dose of 0.01 to 0.04 μg/kg for 1α,25-dihydroxyvitamin $D_3$, or in a dose of 0.01 to 0.1 μg/kg for Compound No. 22. After the last dose, 24-hour urine was sampled and the blood was taken under ether anesthesia. After the animal was euthanized, the lumbar vertebrae were removed.

The lumbar bone mineral density was measured at the third lumbar vertebra site using a dual X-ray bone mineral content measuring device (DCS-600, ALOKA). The blood and urinary calcium concentrations and the urinary creatinine concentration were measured with an automatic analyzer (Model 7170, Hitachi).

Statistical processing was carried out using Statistic Analysis System (SAS) software. A comparison between a sham operation (sham) group and an ovariectomy (OVX) group was made by an unpaired t-test. A comparison between the OVX group and the drug treatment group, or a comparison between the sham group and the drug treatment group was made by Dunnett's multiple test. A P-value less then 5% was considered as a significant difference.

(B) Experimental results

The results of the experiments are shown in FIGS. 3(a) to 3(f). FIGS. 3(a) to 3(c) show the urinary Ca excretion, blood Ca concentration and bone mineral density, respectively, during experiments on treatment with 1α,25-dihydroxyvitamin $D_3$ ($1,25(OH)_2D_3$). The bone mineral density of individual animals was calculated as percentage (%), with the mean bone mineral density for the sham group as 100%.

In the OVX group, the lumbar bone mineral density decreased by about 12%. In the 1α,25-dihydroxyvitamin $D_3$ dose of 0.04 μg/kg, on the other hand, the decrease in bone mineral density was significantly suppressed. However, the 1α,25-dihydroxyvitamin $D_3$ dose of 0.04 μg/kg significantly increased both the blood Ca level and the urinary Ca excretion in comparison with the sham group and the OVX group. Thus, the action on the bone mineral density and the action of increasing the blood Ca concentration and the urinary Ca excretion were simultaneously observed with the 1α,25-dihydroxyvitamin $D_3$.

FIGS. 3(d) to 3(f), on the other hand, show the urinary Ca excretion, blood Ca concentration and bone mineral density, respectively, during experiments on treatment with Compound No. 22. The bone mineral density of individual animals was calculated as percentage (%), with the mean bone mineral density for the sham group as 100%.

In the OVX group, the lumbar bone mineral density decreased by about 11%. In the Compound No. 22 treatment group, on the other hand, a dose-dependent effect of suppressing the decrease in bone mineral density was observed at doses of 0.01 to 0.1 μg/kg and a significant suppressing effect was noted at the dose of 0.1 μg/kg. Unlike la,25-dihydroxyvitamin $D_3$, Compound No. 22 showed no increases in the blood Ca concentration and urinary Ca excretion and exhibited a disparity between the action on the bone mineral density and the action on Ca.

The affinity of Compound No. 22 for a vitamin D receptor was 0.8 times that of la,25-dihydroxyvitamin $D_3$.

Example 3: Effects of Compound No. 22 and 1α-hydroxyvitamin $D_3$ on bone mass decrease in osteoporosis model rats (oral treatment)

(A) Experimental method

8-week-old W-I female rats (Imamichi Institute for Animal Reproduction) were subjected to overiectomy. From the following day onward, 1α-hydroxyvitamin $D_3$ was orally administered 5 times weekly in a daily dose of 0.1 μg/kg, or Compound No. 22 was orally administered daily for 5 weeks in a daily dose of 3 μg/kg given as a single dose or two divided doses. After the last dose, 24-hour urine was sampled and a blood sample was taken under ether anesthesia.

After the animal was euthanized, the lumbar vertebrae were removed. The lumbar bone mineral density was measured at the third lumbar vertebra site using a dual X-ray bone mineral content measuring device (DCS-600EX, ALOKA). The blood and urinary calcium concentrations and the urinary creatinine concentration were measured with an automatic analyzer (Model 7170, Hitachi).

Statistical processing was carried out using basic software for statistical analysis (SAS). A comparison between the sham group and the OVX group, a comparison between the OVX group and the 1α-hydroxyvitamin $D_3$ treatment group, or a comparison between the sham group and the 1α-hydroxyvitamin $D_3$ treatment group was made by an unpaired t-test, with the p value less than 5% representing a significant difference. A comparison between the OVX group and the Compound No. 22 group, or a comparison between the sham group and the Compound No. 22 group was made by Dunnett's multiple test, with the p value less than 5% showing a significant difference.

(B) Experimental results

The results of the experiments are shown in FIGS. 4(a) to 4(c). FIGS. 4(a) to 4(c) show the urinary Ca excretion, blood Ca concentration and bone mineral density, respectively.

In the OVX group, the lumbar bone mineral density decreased significantly. In the 1α-hydroxyvitamin $D_3$ (1α (OH)$D_3$) group receiving 0.1 μg/kg orally, the decrease in bone mineral density by OVX was significantly suppressed, but both the blood Ca level and the urinary Ca excretion significantly increased in comparison with the sham group and the OVX group.

In the group orally administered Compound No. 22 in a dose of 3 μg/kg once daily or in a dose of 1.5 μg/kg twice daily, the effect of suppressing the decrease in bone mineral density was comparable to or higher than that of 1α-hydroxyvitamin $D_3$. Unlike 1α-hydroxyvitamin $D_3$, however, no increases occurred in the blood Ca concentration and urinary Ca excretion and a disparity between the action on the bone mineral density and the action on Ca was observed.

Example 4: Effects of Compound No. 22 on bone strength decrease and bone resorption in osteoporosis model rats (oral treatment)

(A) Experimental method

13-month-old W-I female rats (Imamichi Institute for Animal Reproduction) were subject to overiectomy. From the following day onward, Compound No. 22 was administered p.o. daily for 3 months in a daily dose of 1.5 to 6 μg/kg given as two divided doses. After the last dose, 24-hour urine was sampled and a blood sample was taken under ether anesthesia. After the animal was euthanized, the lumbar vertebrae were removed.

The lumbar bone mineral density was measured at the second to fourth lumbar vertebra site using a dual X-ray bone mineral content measuring device (DCS-600EX, ALOKA). The bone strength of the fifth lumbar vertebra was measured with Autograph AG-2000E (Shimadzu Corp.). The blood calcium concentration and the urinary creatinine concentration were measured with an automatic analyzer (Model 7170, Hitachi). The urinary deoxypyridinoline was measured with Osteolinks DPD.

Statistical Analysis was carried out using Statistic Analysis System (SAS) software. A comparison between the sham group and the OVX group was made by an unpaired t-test. A comparison between the OVX group and the Compound No. 22 treatment group, or a comparison between the sham group and the Compound No. 22 treatment group was made by Dunnett's multiple test, with the p value less than 5% showing a significant difference.

(B) Experimental results

The results of the experiments are shown in FIGS. 5(a) to 5(d). FIGS. 5(a) to 5(d) show the blood Ca concentration, urinary deoxypyridinoline (Dpyr) excretion, bone mineral density and bone strength, respectively.

In any groups receiving Compound No. 22 orally in a dose of 0.75, 1.5 or 3 μg/kg twice daily for 3 months, the decrease in the bone mineral density by OVX was significantly suppressed and the decrease in the bone strength by OVX was also significantly suppressed, without increases in the serum Ca concentration.

Furthermore, the urinary Dpyr excretion increased by OVX was significantly decreased by treatment with Compound No. 22. Thus, the effect of Compound No. 22 on the bone was confirmed to be mediated by a bone resorption suppressing action.

INDUSTRIAL APPLICABILITY

The screening method of the present invention makes it possible to search for a vitamin D derivative which

is targeted at a transcription factor (especially, a transcription factor whose activation is inhibited via a vitamin D receptor) and which can show an effect on the bone without inducing hypercalcemia. The vitamin D derivative found by the screening method of the present invention, in particular, can be expected to show a therapeutic effect on a disease, whose onset or progression the activation of the targeted transcription factor is involved in, by inhibiting the activation of the transcription factor.

## Claims

1. A method for screening a compound having an affinity for a vitamin D receptor, comprising:

   measuring a vitamin D receptor-associated, vitamin D responsive element (VDRE)-mediated transcription promoting activity of a test compound and a vitamin D receptor-associated inhibitory action of the test compound against activity of a transcription factor; and
   selecting a compound which is more potent in inhibitory action against the activity of the transcription factor relative to VDRE-mediated transcription promoting activity.

2. The method according to claim 1, wherein the compound having an affinity for the vitamin D receptor is a vitamin D derivative.

3. The method according to claim 1 or 2, wherein the transcription factor is AP-1 complex or NF-κB family.

4. The method according to any one of claims 1 to 3, wherein the VDRE-mediated transcription promoting activity is measured by a reporter gene assay system.

**5.** The method according to any one of claims 1 to 4, wherein the inhibitory action against the activity of the transcription factor is measured by a reporter gene assay system.

**6.** The method according to any one of claims 1 to 5, wherein a test compound and a standard compound are measured for the VDRE-mediated transcription promoting activity and the inhibitory action on the activity of the transcription factor and the test compound satisfying the following equation:

(a relative value of the inhibitory action of the test compound on the activity of the transcription factor as compared with that of the standard compound)/(a relative value of the VDRE-mediated transcription promoting activity of the test compound as compared with that of the standard compound) $> 1$

is selected as the compound which is relatively more potent in the inhibitory action on the activity of the transcription factor than in the VDRE-mediated transcription promoting activity.

**7.** The method according to claim 6, wherein a test compound satisfying the following equation:

(a relative value of the inhibitory action of the test compound against the activity of the transcription factor as compared with that of the standard compound)/(a relative value of the VDRE-mediated transcription promoting activity of the test compound as compared with that of the standard compound) $\geq 10$

is selected as the compound which is more potent in the inhibitory action against the activity of the transcription factor relative to the VDRE-mediated transcription promoting activity.

**8.** The method according to claim 6 or 7, wherein the standard compound is 1$\alpha$,25-dihydroxyvitamin $D_3$.

**9.** A compound haying an affinity for a vitamin D receptor, **characterized in that** the compound is selected by the method according to any one of claims 1 to 8 and the inhibitory action of the compound against the activity of the transcription factor is stronger than the VDRE-mediated transcription promoting activity of the compound.

**10.** The compound according to claim 9, which is a vitamin D derivative.

**11.** A therapeutic drug for a transcription-factor related disease, the therapeutic drug containing the compound according to claim 9 or 10 and the activity of the transcription factor being inhibited by a vitamin D receptor.

**12.** The therapeutic drug according to claim 11, wherein the transcription-factor related disease is a metabolic bone disease.

**13.** The therapeutic drug according to claim 12, wherein the metabolic bone disease is osteoporosis.

**14.** The therapeutic drug according to any one of claims 11 to 13, wherein the transcription factor is AP-1 complex or

NF-κB family.

**15.** The therapeutic drug according to any one of claims 11 to 14, which is a vitamin D receptor-mediated inhibitor of activity of AP-1 complex or a vitamin D receptor-mediated inhibitor of activity of NF-κB family.

**16.** A kit for the method according to any one of claims 1 to 8, including:

(a) a vector for evaluating the inhibitory action on activity of the transcription factor, said vector containing a binding sequence of the transcription factor and a reporter gene;
(b) a vector for evaluating the VDRE-mediated transcription promoting activity, said vector containing a VDRE sequence and a reporter gene;
(c) if desired, a vitamin D receptor expression vector; and
(d) a reagent for detecting a product of the reporter gene.

**17.** A drug for treatment of osteoporosis, containing as an active ingredient a compound having a value, expressed by the following equation, of more than 1:

(a relative value of the inhibitory action of the test compound against the activity of the transcription factor as compared with that of the standard compound)/(a relative value of the VDRE-mediated transcription promoting activity of the test compound as compared with that of the standard compound),

when the VDRE-mediated transcription promoting activity and the inhibitory action against the activity of the transcription factor are measured by the method according to claim 1.

**18.** The drug for treatment of osteoporosis according to claim 17, containing as the active ingredient the compound having a value, expressed by the following equation, of 10 or more:

(a relative value of the inhibitory action of the test compound on the activity of the transcription factor as compared with that of the standard compound)/(a relative value of the VDRE-mediated transcription promoting activity of the test compound as compared with that of the standard compound).

**19.** The drug for treatment of osteoporosis according to claim 17 or 18, wherein the compound is a vitamin D derivative represented by the following general formula (1):

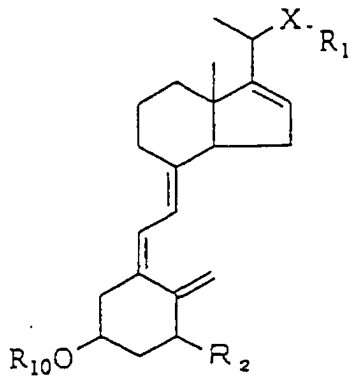

(1)

wherein X represents an oxygen atom or a sulfur atom; $R_1$ represents a saturated or unsaturated aliphatic hydrocarbon group optionally substituted by a hydroxyl group or a protected hydroxyl group or a $-COR_{12}$ group, wherein $R_{12}$ is an alkyl group, an aryl group or an alkoxy group; $R_2$ represents $-OR_9$ or a hydrogen atom; and $R_9$ and $R_{10}$ are the same or different and each represent a hydrogen atom or a protecting group.

**20.** The drug for treatment of osteoporosis according to claim 19, wherein $R_2$ is $-OR_9$.

**21.** The drug for treatment of osteoporosis according to claim 19, wherein $R_1$ is a saturated aliphatic $C_{1-15}$hydrocarbon group optionally substituted by a hydroxyl group.

**22.** The drug for treatment of osteoporosis according to claim 19, wherein $R_1$ is a unsaturated aliphatic $C_{2-15}$hydrocarbon group optionally substituted by a hydroxyl group.

**23.** The drug for treatment of osteoporosis according to claim 19, wherein $R_1$ is a group (2):

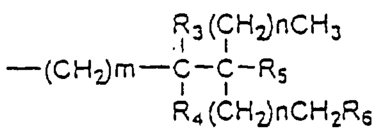

(2)

where $R_3$ and $R_4$ are the same or different and each represent a hydrogen atom or a hydroxyl group, or together have an oxygen atom to represent =O, provided that $R_3$ and $R_4$ are not hydroxyl groups at the same time; $R_5$ and $R_6$ each represent a hydrogen atom or a hydroxyl group, provided that $R_6$ is not a hydroxyl group when $R_3$ or $R_4$ is a hydroxyl group; m is an integer of 1 to 4; and n is an integer of 0 to 2,
or $R_1$ is a group (3):

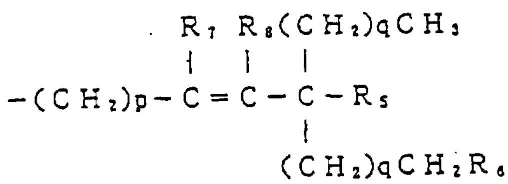

(3)

where $R_5$ and $R_6$ are the same or different and each represent a hydrogen atom or a hydroxyl group; $R_7$ and $R_8$ each represent a hydrogen atom or together represent a covalent bond; p is an integer of 1 to 3; and q is an integer of 0 to 2.

**24.** The drug for treatment of osteoporosis according to claim 19, wherein $R_1$ is a 3-hydroxy-3-methylbutyl group.

**25.** The drug for treatment of osteoporosis according to claim 19, wherein the 20-position of the vitamin D derivative

represented by the general formula (1) is in an S-configuration.

**26.** The drug for treatment of osteoporosis according to claim 19, wherein the 20-position of the vitamin D derivative represented by the general formula (1) is in an R-configuration.

**27.** The drug for treatment of osteoporosis according to claim 19, wherein the vitamin D derivative represented by the general formula (1) is 1,3-dihydroxy-20-(3-hydroxy-3-methylbutylthio)-9,10-secopregna-5,7,10(19),16-tetraene.

**28.** The drug for treatment of osteoporosis according to claim 19, wherein the vitamin D derivative represented by the general formula (1) is 1α,3β-dihydroxy-20(S)-(3-hydroxy-3-methylbutylthio)-9,10-secopregna-5,7,10(19), 16-tetraene.

**29.** The drug for treatment of osteoporosis according to claim 19, wherein the vitamin D derivative represented by the general formula (1) is 1α,3β-dihydroxy-20(R)-(3-hydroxy-3-methylbutylthio)-9,10-secopregna-5,7, 10(19), 16-tetraene.

**30.** The drug for treatment of osteoporosis according to claim 19, wherein the vitamin D derivative represented by the general formula (1) is 1α,3β-dihydroxy-20(R)-((E)-4-hydroxy-4-methyl-2-pentenylthio)-9,10-secopregna-5,7,10 (19),16-tetraene.

**31.** The drug for treatment of osteoporosis according to claim 19, wherein the vitamin D derivative represented by the general formula (1) is, 1α,3β-dihydroxy-20(R)-((E)-4-ethyl-4-hydroxy-2-hexenylthio)-9,10-secopregna-5,7,10(19), 16-tetraene.

**32.** The drug for treatment of osteoporosis according to claim 19, wherein the vitamin D derivative represented by the general formula (1) is 1α,3β-dihydroxy-20(S)-(2-hydroxy-2-methylpropylthio)-9,10-secopregna-5,7,10(19), 16-tetraene.

**33.** The drug for treatment of osteoporosis according to claim 19, wherein the vitamin D derivative represented by the general formula (1) is 1α,3β-dihydroxy-20(R)-(2-hydroxy-2-methylpropylthio)-9,10-secopregna-5,7,10(19), 16-tetraene.

**34.** The drug for treatment of osteoporosis according to claim 19, wherein the vitamin D derivative represented by the general formula (1) is 1α,3β-dihydroxy-20(S)-{2(S)-hydroxy-3-methylbutyloxy}-9,10-secopregna-5,7,10(19), 16-tetraene.

**35.** The drug for treatment of osteoporosis according to claim 19, wherein the vitamin D derivative represented by the general formula (1) is 1α,3β-dihydroxy-20(S)-{2(R)- hydroxy-3-methylbutyloxy}-9,10-secopregna-5,7,10(19), 16-tetraene.

**36.** The drug for treatment of osteoporosis according to claim 19, wherein the vitamin D derivative represented by the general formula (1) is 1α,3β-dihydroxy-20(S)-(2-ethyl-2-hydroxybutylthio)-9,10-secopregna-5,7,10(19), 16-tetraene,

**37.** The drug for treatment of osteoporosis according to claim 19, wherein the vitamin D derivative represented by the general formula (1) is 1α,3β-dihydroxy-20(R)-(2-ethyl-2-hydroxybutylthio)-9,10-secopregna-5,7,10(19), 16-tetraene.

**38.** The drug for treatment of osteoporosis according to claim 19, wherein the vitamin D derivative represented by the general formula (1) is 1α,3β-dihydroxy-20(R)-(4-ethyl-4-hydroxy-2-hexynyloxy)-9,10-secopregna-5,7,10(19), 16-tetraene.

**39.** The drug for treatment of osteoporosis according to claim 19, wherein the vitamin D derivative represented by the general formula (1) is 1α,3β-dihydroxy-20(R)-{3-ethyl-2(S)-hydroxypentylthio)-9,10-secopregna-5,7,10(19), 16-tetraene.

FIG. 1

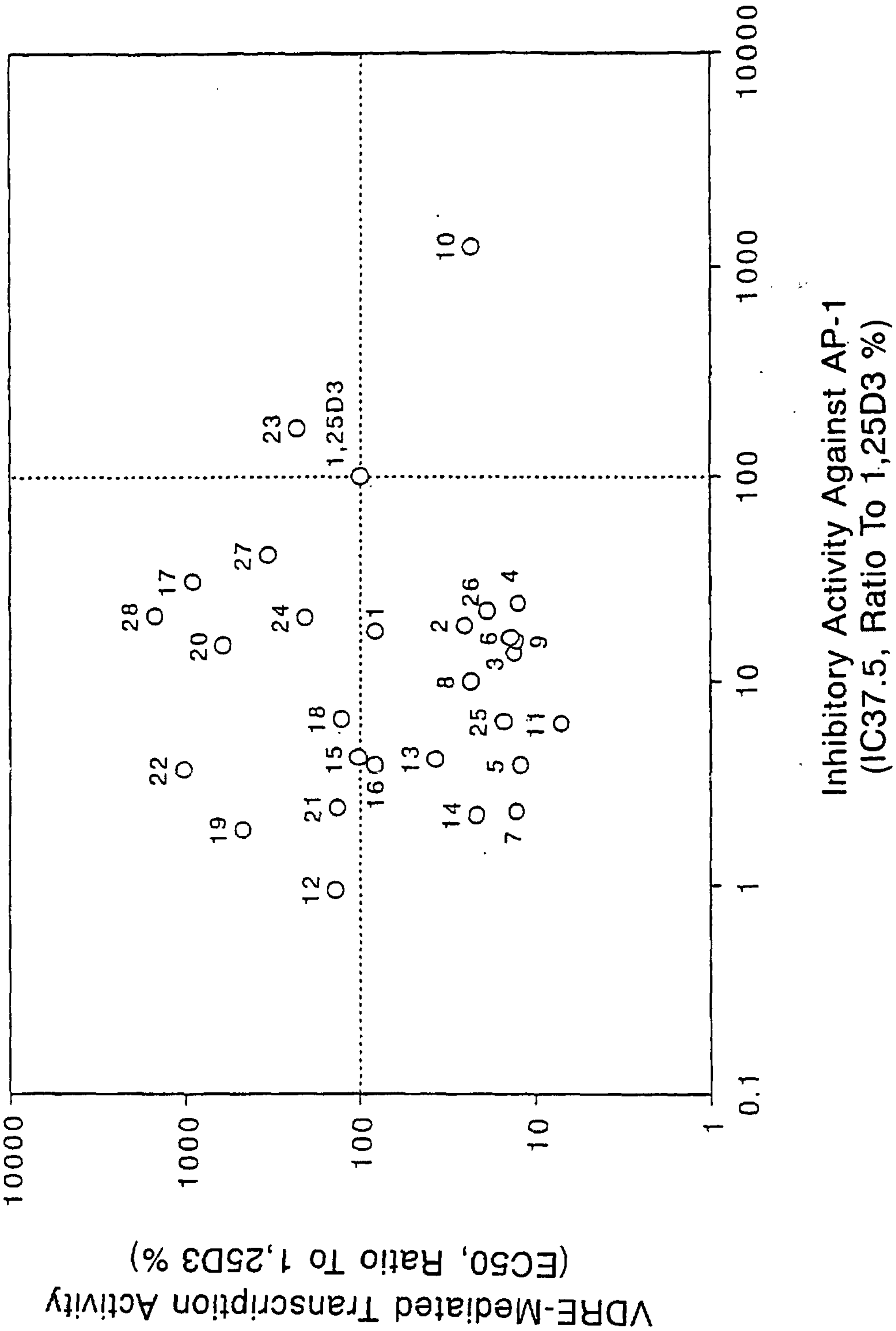
Inhibitory Activity Against AP-1
(IC37.5, Ratio To 1,25D3 %)
VDRE-Mediated Transcription Activity
(EC50, Ratio To 1,25D3 %)
1,25D3

*FIG. 2*

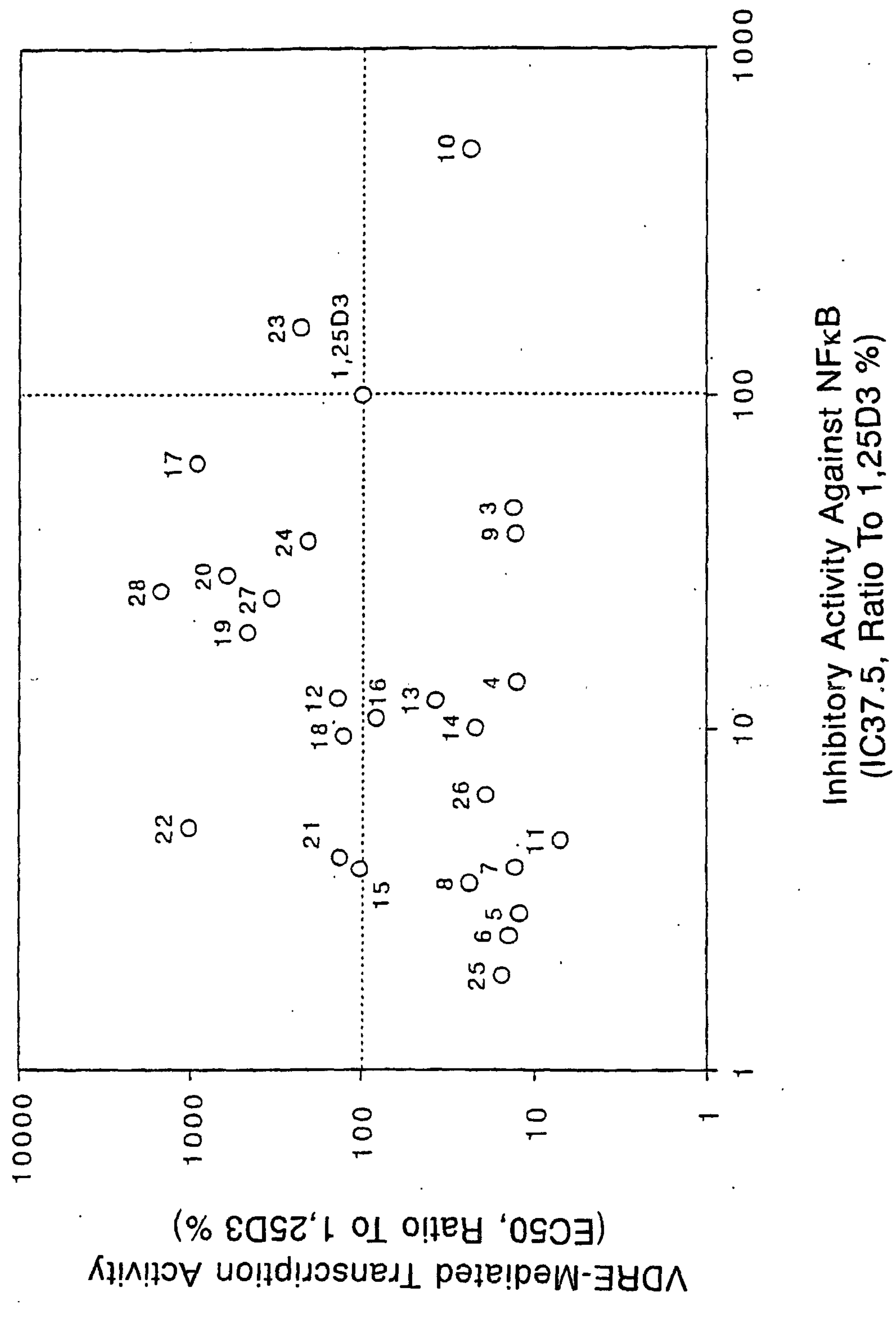
Inhibitory Activity Against NFκB
(IC37.5, Ratio To 1,25D3 %)
VDRE-Mediated Transcription Activity
(EC50, Ratio To 1,25D3 %)
1
10
100
1000
10000
1,25D3
1
2
3
4
5
6
7
8
9
10
11
12
13
14
15
16
17
18
19
20
21
22
23
24
25
26
27
28

FIG. 3

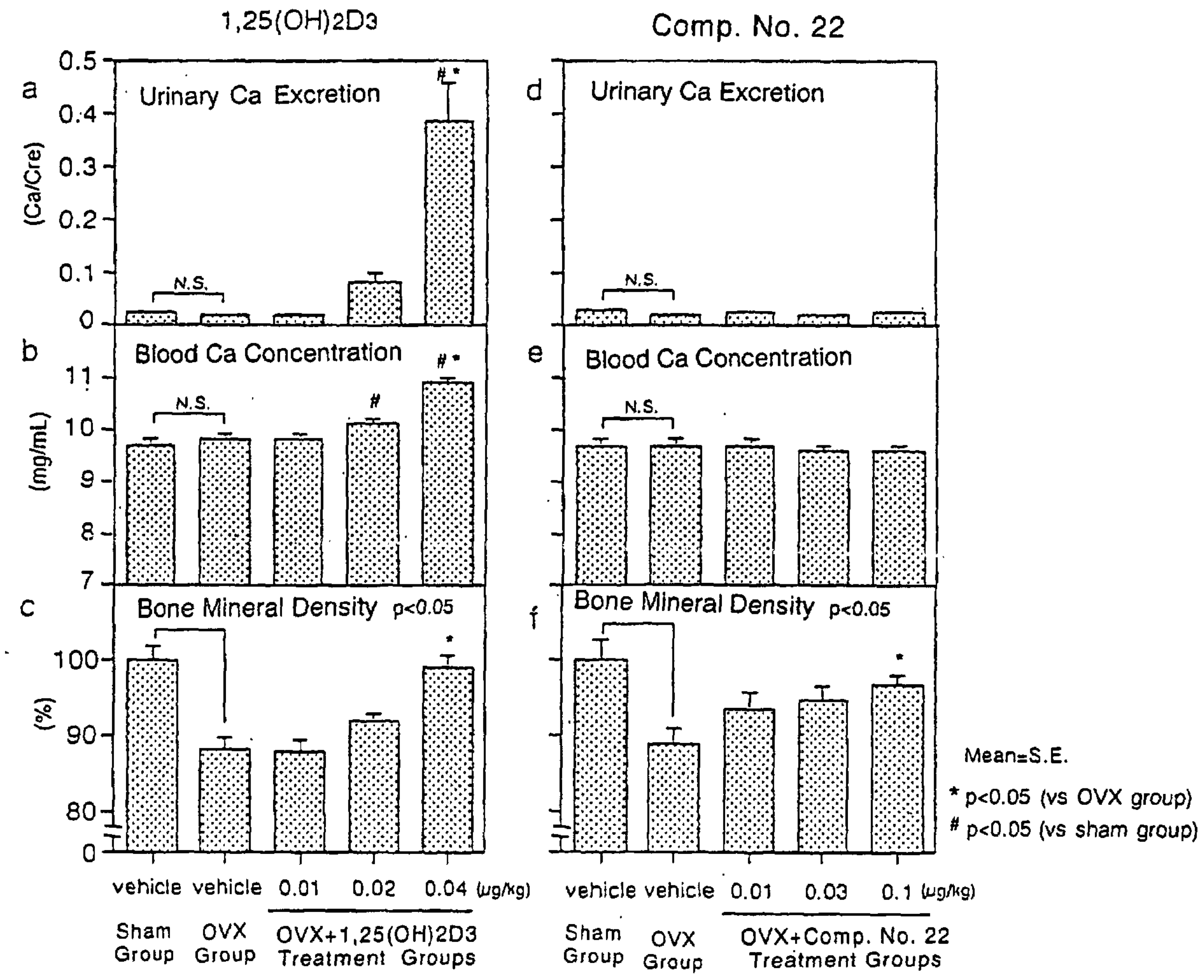
1,25(OH)2D3
Comp. No. 22
a
Urinary Ca Excretion
(Ca/Cre)
0.5
0.4
0.3
0.2
0.1
0
N.S.
#*
b
Blood Ca Concentration
(mg/mL)
11
10
9
8
7
#
#*
c
Bone Mineral Density p<0.05
(%)
100
90
80
*
d
Urinary Ca Excretion
e
Blood Ca Concentration
f
Bone Mineral Density p<0.05
Mean±S.E.
* p<0.05 (vs OVX group)
# p<0.05 (vs sham group)
vehicle vehicle 0.01 0.02 0.04 (µg/kg)
Sham Group
OVX Group
OVX+1,25(OH)2D3 Treatment Groups
vehicle vehicle 0.01 0.03 0.1 (µg/kg)
Sham Group
OVX Group
OVX+Comp. No. 22 Treatment Groups

FIG. 4

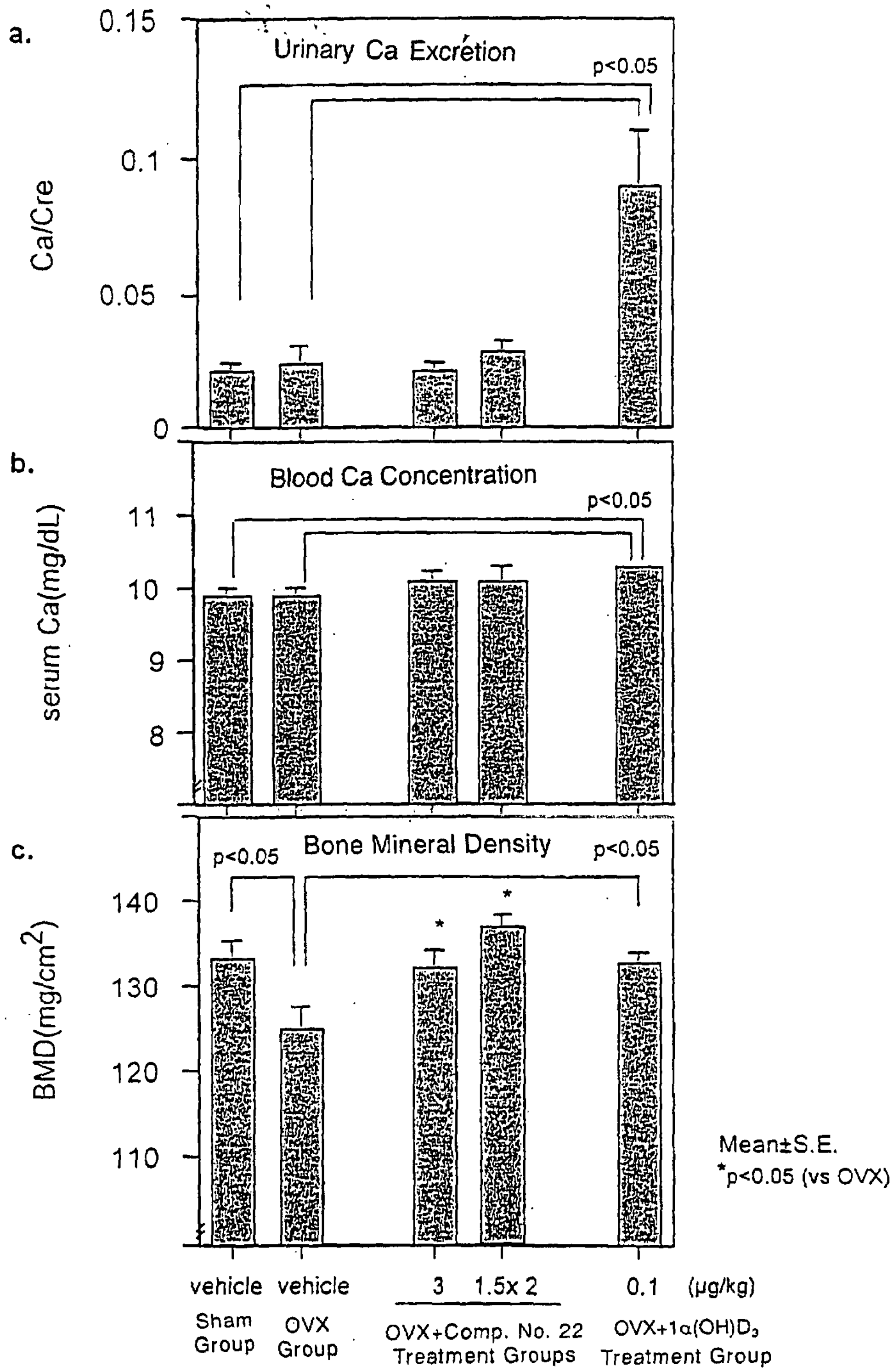
a.
0.15
Urinary Ca Excretion
p<0.05
0.1
Ca/Cre
0.05
0
b.
Blood Ca Concentration
p<0.05
11
10
9
8
serum Ca(mg/dL)
c.
p<0.05
Bone Mineral Density
p<0.05
140
130
120
110
BMD(mg/cm2)
*
*
Mean±S.E.
*p<0.05 (vs OVX)
vehicle
vehicle
3
1.5x 2
0.1
(μg/kg)
Sham Group
OVX Group
OVX+Comp. No. 22 Treatment Groups
OVX+1α(OH)D3 Treatment Group

# FIG. 5

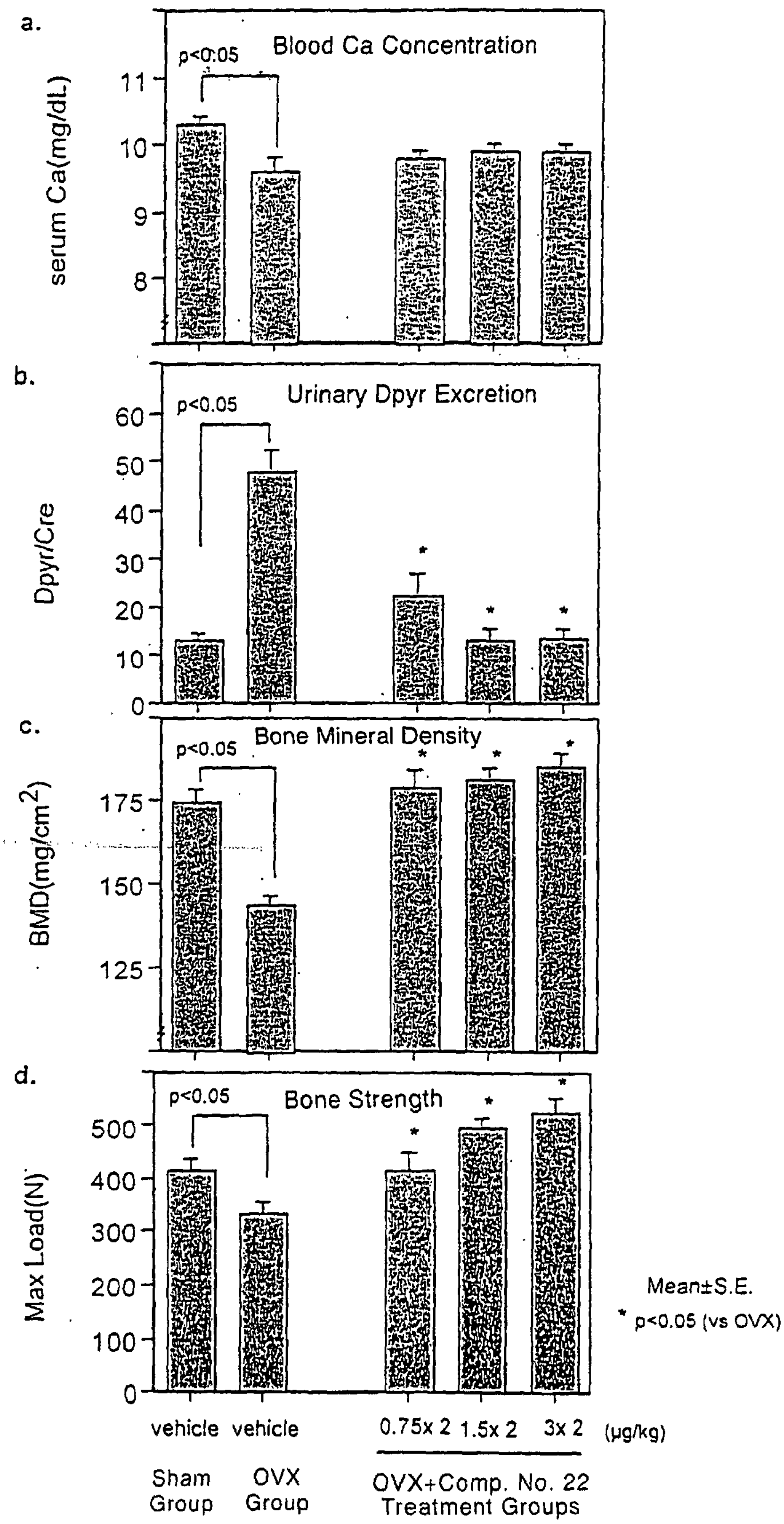
a.
Blood Ca Concentration
p<0.05
serum Ca(mg/dL)
11
10
9
8
b.
Urinary Dpyr Excretion
p<0.05
Dpyr/Cre
60
50
40
30
20
10
0
c.
Bone Mineral Density
p<0.05
BMD(mg/cm2)
175
150
125
d.
Bone Strength
p<0.05
Max Load(N)
500
400
300
200
100
0
vehicle vehicle
0.75x 2 1.5x 2 3x 2 (μg/kg)
Sham Group
OVX Group
OVX+Comp. No. 22 Treatment Groups
Mean±S.E.
* p<0.05 (vs OVX)

## INTERNATIONAL SEARCH REPORT

International application No.
PCT/JP00/00614

**A. CLASSIFICATION OF SUBJECT MATTER**
Int.Cl[7] G01N33/50, G01N33/15, A61K31/59, G01N33/82

According to International Patent Classification (IPC) or to both national classification and IPC

**B. FIELDS SEARCHED**

Minimum documentation searched (classification system followed by classification symbols)
Int.Cl[7] G01N33/50, G01N33/15, A61K31/59, G01N33/82

Documentation searched other than minimum documentation to the extent that such documents are included in the fields searched
Jitsuyo Shinan Koho 1922-1996 Toroku Jitsuyo Shinan Koho 1994-2000
Kokai Jitsuyo Shinan Koho 1971-2000 Jitsuyo Shinan Toroku Koho 1996-2000

Electronic data base consulted during the international search (name of data base and, where practicable, search terms used)
BIOSIS(DIALOG)
WPI(DIALOG)

**C. DOCUMENTS CONSIDERED TO BE RELEVANT**

| Category* | Citation of document, with indication, where appropriate, of the relevant passages | Relevant to claim No. |
|---|---|---|
| Y | WO, 98/28266, A (Chugai Pharmaceutical Co., Ltd.), 02 July, 1998 (02.07.98), Full text & JP, 10-231284, A & EP, 947504, A | 10-15,17-39 |
| Y | EP, 184206, A (Chugai Pharmaceutical Co., Ltd.), 11 June, 1986 (11.06.86), page 1, lines 1 to 10 & JP, 61-267549, A & US, 4666634, A | 10-15,17-39 |
| Y | WO, 98/39292, A (KURARAY CO., LTD.), 11 September, 1998 (11.09.98), page 1, line 13 to page 2, line 16 & JP, 10-245372, A | 10-15,17-39 |
| A | WO, 95/31722, A (LIGAND FARMACEUTICALS INCORPORATED), 23 November, 1995 (23.11.95) & JP, 10-500485, A & EP, 765475, A | 1-8, 10-39 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 February, 2000 (28.02.00) | 07.03.00 |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/00614

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 9
because they relate to parts of the international application that do not comply with the prescribed requirements to such an extent that no meaningful international search can be carried out, specifically:
The subject matter of claim 9 does not define the structure of the compounds.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)